(19) Europäisches Patentamt
European Patent Office
Office européen des brevets

(11) EP 3 038 588 B1

(12) EUROPÄISCHE PATENTSCHRIFT

(45) Veröffentlichungstag und Bekanntmachung des Hinweises auf die Patenterteilung:
**12.12.2018 Patentblatt 2018/50**

(21) Anmeldenummer: **14771769.8**

(22) Anmeldetag: **22.08.2014**

(51) Int Cl.:
***A61K 8/02*** (2006.01) ***A61K 8/22*** (2006.01)
***A61K 8/73*** (2006.01) ***A61Q 5/08*** (2006.01)
***A61Q 5/10*** (2006.01) ***B65D 81/32*** (2006.01)

(86) Internationale Anmeldenummer:
**PCT/DE2014/200420**

(87) Internationale Veröffentlichungsnummer:
**WO 2015/028016 (05.03.2015 Gazette 2015/09)**

(54) **PRODUKTE ZUR OXIDATIVEN FARBVERÄNDERUNG VON KERATINISCHEN FASERN IM SPENDER**

PRODUCTS FOR THE OXIDATIVE COLOUR CHANGING OF KERATIN FIBRES IN A DISPENSER

PRODUITS DE MODIFICATION DE LA COULEUR DE FIBRES DE KÉRATINE EN DISTRIBUTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.08.2013 DE 102013217026**

(43) Veröffentlichungstag der Anmeldung:
**06.07.2016 Patentblatt 2016/27**

(73) Patentinhaber: **Henkel AG & Co. KGaA**
**40589 Düsseldorf (DE)**

(72) Erfinder:
- **NEUBA, Constanze**
  **41516 Grevenbroich (DE)**
- **SCHETTIGER, Norbert**
  **40723 Hilden (DE)**
- **MÜLLER, Burkhard**
  **40221 Düsseldorf (DE)**

(56) Entgegenhaltungen:
**EP-A2- 1 669 104** **WO-A1-03/089330**
**DE-A1-102008 017 104**

- **J. SCHMUCKER-CASTNER ET AL: "Rheology Modification of Hydrogen Peroxide-based Applications Using Cross-linked Polyacrylic Acid Polymers", INTERNATIONAL JOURNAL OF COSMETIC SCIENCE, Bd. 21, Nr. 5, 1. Oktober 1999 (1999-10-01), Seiten 313-325, XP055157322, ISSN: 0142-5463, DOI: 10.1046/j.1467-2494.1999.198475.x**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft ein Produkt zur Färbung und/oder Aufhellung von keratinischen Fasern, welches einen Spender mit zwei voneinander getrennten Kammern umfasst, die eine gemeinsame Auslassöffnung besitzen, wobei jede der beiden Kammern jeweils eine Zubereitung mit bestimmten verdickenden Polymeren in speziellen Gewichtsverhältnissen beinhaltet. Weiterhin betrifft die vorliegende Erfindung ein Verfahren, bei dem keratinische Fasern unter Anwendung dieses Produktes gefärbt und/oder aufgehellt werden.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten.

Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln - in der Regel Wasserstoffperoxid - untereinander die eigentlichen Farbstoffe ausbilden. Oxidationsfärbemittel zeichnen sich durch hervorragende, lang anhaltende Färbeergebnisse aus.

Die reine Aufhellung oder Blondierung von Haaren erfolgt oft durch Einsatz von Oxidationsmitteln ohne den Zusatz von Oxidationsfarbstoffvorprodukten. Für einen mittleren Blondiereffekt genügt der Einsatz von Wasserstoffperoxid als Oxidationsmittel allein, für das Erzielen eines stärkeren Blondiereffektes wird üblicherweise eine Mischung aus Wasserstoffperoxid und Peroxidisulfatsalzen eingesetzt.

Oxidative Farbveränderungsmittel kommen üblicherweise in Form von Zweikomponenten-Mitteln auf den Markt, bei welchen die beiden Komponenten in zwei getrennten Containern separat konfektioniert vorliegen und kurz vor der Anwendung miteinander vermischt werden müssen.

Bei der ersten Komponente handelt es sich in der Regel um eine alkalisch eingestellte Formulierung, die oft in Form einer Creme oder eines Gels vorliegt und welche, sofern gleichzeitig mit der Aufhellung auch eine Farbänderung gewünscht wird, zusätzlich auch die Oxidationsfarbstoffvorprodukte enthält. Diese erste Komponente wird in den meisten Fällen in einer Tube, seltener in einem Kunststoff- oder Glascontainer bereitgestellt.

Bei der zweiten Komponente handelt es sich um eine - aus Stabilitätsgründen in der Regel sauer eingestellte - Formulierung, welche als Oxidationsmittel Wasserstoffperoxid in Konzentrationen von 3 bis 12 Gew.-% enthält. Die Oxidationsmittelformulierung liegt meist in Form einer Emulsion oder Dispersion vor und wird in der Regel in einer Kunststoff-Flasche mit wiederverschließbarer Auslassöffnung zur Verfügung gestellt (Entwicklerflasche).

Zur Herstellung der anwendungsbereiten Mischung muss der Verbraucher beide Komponenten kurz vor der Anwendung miteinander vermischen. Hierfür wird normalerweise die alkalische Creme- oder Gelkomponente aus der Tube bzw. dem Glas- oder Kunststoffcontainer komplett in die Entwicklerflasche überführt, dann werden beide Komponenten durch Schütteln möglichst vollständig und homogen miteinander vermischt und schließlich über eine Auslassöffnung im Kopf der Entwicklerflasche entnommen.

Dieser separate Mischungsvorgang hat für den Konsumenten jedoch eine Reihe von Nachteilen. So kann durch die unvollständige Entleerung der Tube das Mengenverhältnis beider Komponenten verändert werden, was zu Abweichungen im gewünschten Farbergebnis führt. Bei zu kurzem Verschütteln bzw. Vermischen beider Komponenten ist die Anwendungsmischung inhomogen, die Folge hiervon ist ein ungleichmäßiges Farbergebnis. Darüber hinaus ist es auch aus Gründen des Anwendungskomforts wünschenswert, auf diesen Mischungsschritt komplett zu verzichten.

Zur Vermeidung dieser Nachteile wurden Mehrkammerbehältnisse mit gemeinsamer Austrittsöffnung entwickelt, bei denen die beiden Komponenten im Ventil oder Spender während des Austritts gemischt werden. Die Entnahme der Anwendungsmischung über den Spender macht ein Vermischen der Komponenten durch den Verbraucher überflüssig und hat den Anwendungskomfort deutlich erhöht.

Doch auch bei diesen Spendern besteht nach wie vor das Problem, dass das Mischungsverhältnis beider Komponenten sich je nach Füllhöhe der Formulierungen in den Kammern, Fließverhalten der Formulierungen und je nach Druck innerhalb des Spenders ändern kann. Dies birgt die große Gefahr, dass sich die Zusammensetzung der Anwendungsmischung im Verlauf der Entnahme ändert. Die damit verbundenen Abweichungen im Farbergebnis sind vom Verbraucher im höchsten Maße unerwünscht.

Bislang ist es nicht gelungen, oxidative Haarfarbänderungsprodukte auf Basis eines Mehrkammersystems bereit zu stellen, die eine Dosierung der Anwendungsmischung in konstanter, definierter Zusammensetzung ermöglichen. In der DE 10 2007 056 935 A1 wurde versucht, die Konstanz des Mischungsverhältnisses durch Entwicklung von Spendern mit speziellem Steuermechanismus zu gewährleisten. Dieser Mechanismus schließt jedoch Fehlbedienungen von Seiten des Verbrauchers nicht aus.

Es war daher die Aufgabe der vorliegenden Erfindung, ein neues Produkt zum oxidativen Färben und/oder Aufhellen von keratinischen Fasern bereit zu stellen, welches auf einem Spender basiert, der die Entnahme der fertigen Anwendungsmischung ermöglicht. Hierbei soll die über den Spender entnommene Anwendungsmischung hinsichtlich ihrer Zusammensetzung definiert und konstant sein und sich nicht in Abhängigkeit vom Befüllungsgrad des Spenders ändern.

Überraschenderweise wurde nun gefunden, dass es möglich ist, eine konstant zusammengesetzte Anwendungsmischung aus einem Mehrkammerspender zu entnehmen, wenn die beiden im Mehrkammerspender befindlichen Zubereitungen hinsichtlich ihres Gehaltes und ihrer Zusammensetzung an verdickenden Polymeren genau aufeinander abgestimmt sind.

Ein erster Gegenstand der Erfindung ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0 liegt und
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, enthält und
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, enthält.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Die in den Kammern (A) und (B) befindlichen Zubereitungen (a) und (b) enthalten die wesentlichen Inhaltsstoffe jeweils in einem kosmetischen Träger, bevorzugt in einem geeigneten wässrigen, alkoholischen oder wässrig-alkoholischen Träger. Zum Zwecke der oxidativen Farbänderung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei den Zubereitungen (a) und/oder (b) um Gele.

Kennzeichnend für die Zubereitungen (a) und (b) ist ihr genau aufeinander abgestimmter Gehalt an verdickenden Polymeren. Unter "verdickenden Polymeren" sind im Sinne der vorliegenden Erfindung organische polymere Verbindungen zu verstehen, die, wenn sie in einer Menge von 0,1 Gew.-% in Wasser gegeben werden (bei 22 °C und einem Druck von 760 mmHg), die Viskosität des Wassers erhöhen. Die Messung der Viskosität erfolgt hierbei bei 22 °C (22 °C / Brookfield-Viskosimeter / Spindel 5 / 4 Upm).

Unter polymeren Verbindungen werden im Sinne der vorliegenden Erfindung auf Wiederholungseinheiten basierende Verbindungen mit einem Molgewicht von mehr als 5000 g/mol, bevorzugt von mehr als 10 000 g/mol und besonders bevorzugt von mehr als 20 000 g/mol verstanden. Das maximale Molgewicht der Polymere ist von den Synthesebedingungen, der Ansatzgröße und dem Polymerisationsgrad abhängig und überschreitet vorzugsweise nicht mehr als $10^8$ g/mol.

Auf Wiederholungseinheiten basierende Verbindungen besitzen mindestens 50 Wiederholungseinheiten, bevorzugt mindestens 100 Wiederholungseinheiten und besonders bevorzugt mindestens 500 Wiederholungseinheiten.

Die Zubereitung (a), welche sich in der Kammer (A) des Spenders befindet, enthält in einem kosmetischen Träger einen oder mehrere der genannten verdickenden Polymere in einer Gesamtgewichtsmenge (G1) von 0,1 bis 40 Gew.-%- bezogen auf das Gesamtgewicht der Zubereitung (a). Die Zubereitung (b), welche sich in der Kammer (B) des Spenders befindet, enthält in einem kosmetischen Träger einen oder mehrere der genannten verdickenden Polymere in einer Gesamtgewichtsmenge (G2) von 0,1 bis 40 Gew.-%-bezogen auf das Gesamtgewicht der Zubereitung (b). Hierbei ist ein erfindungswesentliches Merkmal des Produktes, dass das das Gewichtsverhältnis G1/G2 bei einem Wert von 0,5 bis 2,0 liegt.

Unter der Gesamtgewichtsmenge G1 wird das Gesamtgewicht aller in der Formulierung (a) enthaltenen verdickenden Polymere verstanden. Berechnungsgrundlage ist hierbei das Gesamtgewicht der Zubereitung (a).

Unter der Gesamtgewichtsmenge G2 wird das Gesamtgewicht aller in der Formulierung (b) enthaltenen verdickenden Polymere verstanden. Berechnungsgrundlage ist hierbei das Gesamtgewicht der Zubereitung (b).

Wenn das Gewichtsverhältnis G1/G2 der in den Formulierungen (a) und (b) enthaltenen verdickenden Polymere innerhalb des Wertebereichs von 0,5 bis 2,0 liegt, lassen sich beide Formulierungen reproduzierbar und konstant und über die Austrittsöffnung des Spenders (C) dosieren, d.h. es wird die Entnahme von definierten und konstanten Mengen der Formulierungen (a) und (b) über die gesamte Anwendungsdauer möglich. Auf diese Weise lässt sich über den Spender eine aus (a) und (b) hergestellte Anwendungsmischung entnehmen, die bei jedem Entnahmeschritt die gleiche Zusammensetzung besitzt, unabhängig davon, ob der Spender noch voll gefüllt oder bereits teilweise entleert ist. Hierbei erfolgt die Dosierung umso genauer, je näher das Gewichtsverhältnis G1/G2 bei dem Wert von 1 liegt.

Ein ganz besonders bevorzugtes Produkt zur oxiativen Färbung und/oder Aufhellung keratinischer Fasern ist daher dadurch gekennzeichnet, dass das Gewichtsverhältnis von G1/G2 bei einem Wert von 0,55 bis 1,8, weiter bevorzugt von 0,6 bis 1,6 und besonders bevorzugt von 0,7 bis 1,4 liegt.

Wenn die Zubereitungen (a) und (b) beide eine oder mehrere der genannten verdickenden Polysaccharide enthalten, kann die Viskosität der beiden Zubereitungen besonders gut aufeinander abgestimmt werden. Insbesondere bleibt die Viskosität der beiden Zubereitungen auch über längere Lagerzeiträume stabil.

Überraschenderweise hat sich herausgestellt, dass sich durch den Einsatz der genannten Polysacchariden die rheologischen Eigenschaften der Zubereitungen (a) und (b) besonders gut aufeinander abstimmen lassen. Mit anderen Worten können Zubereitungen (a) und (b), die beide jeweils mindestens ein spezielles Polysacccharid enthalten, wobei das Gesamtgewichtsverhältnis der verdickenden Polymere G1/G2 bei einem Wert von 0,5 bis 2,0 liegt, aus dem Spender über die gesamte Anwendungsdauer in einer besonders konstanten Zusammensetzung entnommen werden. Somit besitzen die beiden Zubereitungen (a) und (b), obwohl sie ansonsten hinsichtlich ihres pH-Wertes und ihrer Inhaltsstoffe stark voneinander differieren können, bei Zusatz von anionischen Polyssachariden nahezu identische rheologische Eigenschaften, die insbesondere auch vom Druck innerhalb des Spenders sowie vom Befüllungsgrad des Spenders unabhängig sind.

Die Bezeichnung Polysaccharide ist die Sammelbezeichnung für makromolekulare Kohlenhydrate, deren Moleküle aus einer großen Zahl glycosidisch miteinander verknüpfter Monosaccharid-Moleküle bestehen (mit einer Molmasse von mind. 5000 g/mol).

Als Polysaccharide werden hier Xanthan Gum, Alginate und Carboxyalkylcellulosen wegen ihrer ganz besonderen Eignung eingesetzt.

Xanthan ist ein Polysaccharid, welches unter anderem aus den Strukturbestandteilen D-Glucose, D-Mannose, D-Glucuronsäure, Acetat und Pyruvat aufgebaut ist und das auch unter dem INCI Namen Xanthan Gum bekannt ist. Xanthan trägt Carboxygruppen und ist anionisch bzw. anionisierbar. Auch die phyiologisch verträglichen Salze von Xanthan sind erfindungsgemäß.

Als Alginate (INCI-Name Algin) werden die Salze der Alginsäure bezeichnet. Alginate sind saure, Carboxy-Gruppen enthaltende Polysaccharide, bestehend aus D-Mannuronsäure und D-Guluronsäure in unterschiedlichen Verhältnissen, welche mit 1-4-glycosidischen Bindungen verknüpft sind. Erfindungsgemäß sind insbesondere die Alkali- als auch die Erdalkalisalze der Alignsäuren. Besonders vorteilhaft hat sich der Einsatz von Alginsäure, Natriumalginat, Kaliumalginat, Ammoniumalginat und/oder Calciumalginat in den erfindungsgemäßen Zubereitungen herausgestellt.

Carboxyalkylcellulosen sind Celluloseether, bei denen die Wasserstoffatome der Hydroxygruppen der Cellulose teilweise oder vollständig durch Carboxyalkylgruppen substituiert sind. Eine bevorzugte Carboxyalkylcellulose ist die Carboxymethylcellulose, die vorzugsweise in Form ihres Natriumsalzes (Natrium-Carboxymethylcellulose) als anionisches Polymer Einsatz finden kann.

Das beanspruchte Produkt zur oxidativen Färbung und/oder Aufhellung keratinischer Fasern, umfasst daher

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
dadurch gekennzeichnet, dass
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält,
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält und
- das Gewichtsverhältnis G1/G2 bei einem Wert von 0,5 bis 2,0 liegt.

Besonders bevorzugt ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
dadurch gekennzeichnet, dass
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält,
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält und
- das Gewichtsverhältnis G1/G2 bei einem Wert von 0,55 bis 1,8 liegt.

Besonders bevorzugt ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält,
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält und
- das Gewichtsverhältnis G1/G2 bei einem Wert von 0,6 bis 1,6 liegt.

Besonders bevorzugt ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält,
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, besonders bevorzugt Xanthan Gum und/oder dessen physiologisch verträgliche Salze, enthält und
- das Gewichtsverhältnis G1/G2 bei einem Wert von 0,7 bis 1,4 liegt.

Neben den vorgenannten besonders bevorzugten Polysacchariden können die Zubereitungen (a) und (b) zusätzlich auch noch weitere verdickende Polymere enthalten. Auch hierbei gilt jedoch die Vorraussetzung, dass die Gesamtgewichtsmenge aller in der Zubereitung (a) enthaltenden verdickenden Polymere (G1) 0,1 bis 40 Gew.-% beträgt und die Gesamtgewichtsmenge aller in der Zubereitung (b) enthaltenden verdickenden Polymere (G2) 0,1 bis 40 Gew.-% beträgt.

In einer weiteren Ausführungsform kann es besonders bevorzugt sein, wenn die Zubereitungen (a) und (b) ein oder mehrere verdickende Polymere aus der Gruppe der Carboxymethylcellulosen, der Alginsäuren und Xanthan Gum enthalten und darüber hinaus kein weiteres verdickendes Polymer enthalten.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Carboxymethylcellulose und/oder deren physiologisch verträgliches Salz enthält und
- die Zubereitung (b) als verdickendes Polymer Carboxymethylcellulose und/oder deren physiologisch verträgliches Salz enthält.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Alginsäure und/oder deren physiologisch verträgliches Salz enthält und
- die Zubereitung (b) als verdickendes Polymer Alginsäure und/oder deren physiologisch verträgliches Salz enthält.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Xanthan Gum und/oder dessen physiologisch verträgliches Salz enthält und
- die Zubereitung (b) als verdickendes Polymer Xanthan Gum und/oder dessen physiologisch verträgliches Salz enthält.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Carboxymethylcellulose und/oder deren physiologisch verträgliches Salz enthält,
- die Zubereitung (b) als verdickendes Polymer Carboxymethylcellulose und/oder deren physiologisch verträgliches Salz enthält und
- und die Zubereitungen (a) und (b) neben Carboxymethylcellulose und/oder deren physiologisch verträglichem Salz keine weiteren verdickenden Polymere enthalten.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Alginsäure und/oder deren physiologisch verträgliches Salz enthält,
- die Zubereitung (b) als verdickendes Polymer Alginsäure und/oder deren physiologisch verträgliches Salz enthält und
- und die Zubereitungen (a) und (b) neben Alginsäure und/oder deren physiologisch verträglichem Salz keine weiteren verdickenden Polymere enthalten.

Bevorzugt ist auch ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0, weiter bevorzugt von 0,55 bis 1,8, noch weiter bevorzugt von 0,6 bis 1,6 und besonders bevorzugt von 0,7 bis 1,4 liegt,
- die Zubereitung (a) als verdickendes Polymer Xanthan Gum und/oder dessen physiologisch verträgliches Salz enthält,
- die Zubereitung (b) als verdickendes Polymer Xanthan Gum und/oder dessen physiologisch verträgliches Salz enthält und
- und die Zubereitungen (a) und (b) neben Xanthan Gum und/oder dessen physiologisch verträglichem Salz keine weiteren verdickenden Polymere enthalten.

Die Zubereitungen (a) und (b) können als verdickendes Polymer auch mindestens ein Acrylsäurepolymer und/oder mindestens ein Methacrylsäurepolymer enthalten. Unter einem Acrylsäurepolymer wird ein Homopolymer der Acrylsäure oder ein Homopolymer eines Acrylsäurederivates verstanden. Unter einem Methacrylsäurepolymer wird ein Homopolymer der Methacylsäure oder ein Homopolymer eines Methacrylsäurederivates verstanden.

Bevorzugte Acrylsäure- und Methacrylsäurepolymere enthalten als Wiederholungseinheiten Strukturen der Formel (P-I),

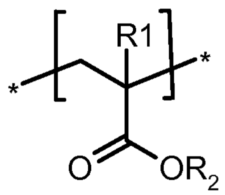

(P-I)

worin

R1 für ein Wasserstoffatom oder eine Methylgruppe steht und

R2 für ein Wasserstoffatom, für eine $C_1$-$C_{30}$-Alkylgruppe, eine Hydroxy-$C_2$-$C_{30}$-alkylgruppe, eine $C_1$-$C_{30}$-Alkoxy-$C_2$-$C_6$-alkylgruppe, für eine $C_1$-$C_{30}$-Alkyl-$(CH_2$-$CH_2$-$O)_n$-O-Gruppe, eine $C_1$-$C_6$-Dialkylamino-$C_2$-$C_6$-alkylgruppe oder für Natrium, Kalium, ½ Magnesium oder ½ Calcium steht,

n für eine Zahl von 1 bis 30 steht.

Im Fall der bevorzugten Acrylsäurepolymere steht R1 für ein Wasserstotfatom, im Fall der bevorzugten Methacrylsäurepolymere steht R1 für eine Methylgruppe.

Bei einem bevorzugten Homopolymer der Acrylsäure handelt es sich um Polyacrylsäure, wie sie beispielsweise von der Firma 3V Sigma unter den Handelsnamen Synthalen K oder Synthalen M oder von der Firma Lubrizol unter den Handelsnamen Carbopol (beispielsweise Carbopol 980, 981, 954, 2984 und 5984), jeweils mit der INCI-Bezeichnung Carbomer, erhältlich ist. Auch das von der BASF vertriebene unter dem Handelsnamen Cosmedia SP (INCI Name: SODIUM POLYACRYLATE) kann in diesem Zusammenhang als bevorzugtes Acrylsäure Homopolymer genannt werden.

Weiterhin können die Zubereitungen (a) und (b) als verdickendes Polymer auch mindestens ein Acrylsäurecopolymer und/oder ein Methacrylsäurecopolymer enthalten.

Unter Acrylsäurecopolymeren werden Copolymere verstanden, die durch Polymerisation von Acrylsäure bzw. Acrylsäurederivaten mit einem oder mehreren von Acrylsäure bwz. Acrylsäurederivaten verschiedenen Monomeren hergestellt werden. Unter Methacrylsäurecopolymeren werden Copolymere verstanden, die durch Polymerisation von Methacrylsäure bzw. Methacrylsäurederivaten mit einem oder mehreren von Methacrylsäure bzw. Methacrylsäurederivaten verschiedenen Momomeren hergestellt werden.

Bevorzugte Acrylsäure- und Methacrylsäurecopolymere enthalten als Wiederholungseinheiten Strukturen der

Formeln (P-II) und (P-III)

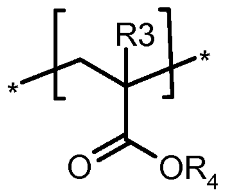 (P-II) 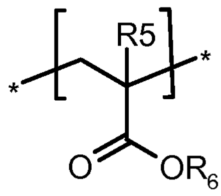 (P-III)

worin

R3, R5 unabhängig voneinander für ein Wasserstoffatom oder eine Methylgruppe stehen,

R4 für ein Wasserstoffatom, für eine $C_1$-$C_{30}$-Alkylgruppe, eine Hydroxy-$C_2$-$C_{30}$-alkylgruppe, eine $C_1$-$C_{30}$-Alkoxy-$C_2$-$C_6$-alkylgruppe, für eine $C_1$-$C_{30}$-Alkyl-$(CH_2$-$CH_2$-$O)_n$-O Gruppe, eine $C_1$-$C_6$-Dialkylamino-$C_2$-$C_6$-alkylgruppe oder für Natrium, Kalium, ½ Magnesium oder ½ Calcium steht,

R6 für ein Wasserstoffatom, für eine $C_1$-$C_{30}$-Alkylgruppe, eine Hydroxy-$C_2$-$C_{30}$-alkylgruppe, eine $C_1$-$C_{30}$-Alkoxy-$C_2$-$C_6$-alkylgruppe, für eine $C_1$-$C_{30}$-Alkyl-$(CH_2$-$CH_2$-$O)_m$-O Gruppe, eine $C_1$-$C_6$-Dialkylamino-$C_2$-$C_6$-alkylgruppe oder für Natrium, Kalium, ½ Magnesium oder ½ Calcium steht,

n für eine Zahl von 1 bis 30 steht,

m für eine Zahl von 1 bis 30 steht.

Bevorzugt sind R3 und R5 voneinander verschieden und/oder R4 und R6 sind voneinander verschieden.

Bevorzugte Acrylsäure- und Methyarcylsäurecopolymere sind die unter der Bezeichnung Simulgel®EG als Compound mit Isohexadecan und Polysorbat-80 vertriebenen Natriumacrylat / Natriumacryloyldimethyltaurat-Copolymere (INCI-Name: SODIUM ACRYLATE/SODIUM ACRYLOYLDIMETHYL TAURATE COPOLYMER, ISOHEXADECANE, POLYSORBATE 80).

Ein in diesem Zusammenhang weiterhin geeignetes Polymer ist das unter der INCI Bezeichnung Acrylates / C10-30 Alkyl Acrylate Crosspolymer bekannte Polymer, das unter dem Handelsnamen Carbopol 1382 von der Firma Noveon erhältlich ist. Ein weiterhin geeignetes Polymer ist das unter der INCI-Bezeichnung Acrylates / Steareth-20 Methacrylate Crosspolymer bekannte Polymer, welches beispielsweise mit dem Handelsnamen Aculyn ® 88 von der Firma Rohm & Haas in Form einer 28 bis 30 Gew.-%-igen Dispersion in Wasser vertrieben wird. Ferner können Polymere nach der INCI-Nomenklatur als Acrylates/Palmeth-25 Acrylate Copolymer oder Acrylates / Palmeth-20 Acrylate Copolymer eingesetzt werden. Solche Polymere sind beispielsweise unter der Handelsbezeichnung Synthalen® W 2000 als 30 bis 32 Gew.-%-ige Emulsion in Wasser von der Firma 3 V Sigma erhältlich.

Es kann ebenfalls bevorzugt sein, als Acrylsäure und/oder Methacrylsäurecopolmyer ein Copolymer aus mindestens einem anionischen Acrylsäure bzw. Methacrylsäure-Monomer und mindestens einem nichtionogenen Monomer einzusetzen. Bevorzugt nichtionogene Monomere sind in diesem Zusammenhang Acrylamid, Methacrylamid, Acrylsäureester, Methacrylsäureester, Vinylpyrrolidon, Vinylether und Vinylester.

Weitere bevorzugte anionische Copolymere sind beispielsweise Copolymere aus Acrylsäure und/oder Methacrylsäure und deren $C_1$-$C_6$-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugt sind aber auch Copolymere aus Acrylsäure und/oder Methacrylsäure, den $C_1$-$C_6$-Alkylestern von Acrylsäure und/oder Methacacrylsäure sowie den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 (INCI-Name: Acrylates/Steareth-20 Methacrylate Copolymer) vertrieben.

Weitere bevorzugte Acrylsäure bzw. Methacrylsäure Copolymere sind Acrylsäure-Acrylamid-Copolymere.

Auch die nachfolgenden zwitterionische Acrylsäure- bzw. Methacrylsäurepolymere können in den Zubereitungen (a) und (b) enthalten sein:

- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,

- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten bevorzugten verdickenden Polymere können in den erfindungsgemäßen Zubereitungen (a) und (b) enthalten sein, wobei wieder die Vorraussetzung gilt, dass die das Verhältnis der Gesamtgewichtsmengen der verdickenden Polymeren (G1) und (G2) in den Zubereitungen (a) und (b) bei einem Wert von 0,5 bis 2,0 liegt.

Im Zuge der zu dieser Erfindung führenden Arbeiten hat sich herausgestellt, dass es sich besonders vorteilhaft auswirkt, wenn die verdickenden Polymere in den Zubereitungen (a) und (b) jeweils in bestimmten Mengenbereichen eingesetzt werden.

Hierbei konnte die vollständigste Entleerbarkeit des Spenders über die Auslassöffnung (C) insbesondere dann gewährleistet werden, wenn die Gesamtgewichtsmengen (G1) und (G2) der verdickenden Polymere in den Zubereitungen (a) und (b) jeweils in einem Mengenbereich zwischen 0,8 und 2,5 Gew.-% lagen. Wurden die Gesamtgewichtsmengen (G1) und (G2) zu hoch gewählt (z.B. oberhalb von 9,5 Gew.-%), kam es zu Verklumpungen innerhalb des Spenders oder während der Entnahme. Wurden die Gesamtgewichtsmengen (G1) und (G2) dagegen zu gering gewählt, war die Anpassung der rheologischen Eigenschaften der beiden Zubereitungen (a) und (b) nur unzureichend möglich, so dass sich die Zusammensetzung der Anwendungsmischung (aus (a) und (b)) im Verlauf des Anwendeprozesses (d.h. bei zunehmender Entleerung des Spenders) veränderte.

Ein besonders bevorzugtes erfindungsgemäßes Produkt ist daher dadurch gekennzeichnet, dass

- die Zubereitung (a) das oder die verdickenden Polymere in einer Gesamtgewichtsmenge (G1) von 0,2 bis 9,5 Gew.-%, bevorzugt von 0,4 bis 7,5 Gew.-%, weiter bevorzugt von 0,6 bis 5,5 Gew.-% und besonders bevorzugt von 0,8 bis 2,5 Gew.-% - bezogen auf das Gesamtgewicht der Zubereitung (a)
- enthält und
- die Zubereitung (b) das oder die verdickende Polymere in einer Gesamtgewichtsmenge (G2) von 0,2 bis 9,5 Gew.-%, bevorzugt von 0,4 bis 7,5 Gew.-%, weiter bevorzugt von 0,6 bis 5,5 Gew.-% und besonders bevorzugt von 0,8 bis 2,5 Gew.-% - bezogen auf das Gesamtgewicht der Zubereitung (b)
- enthält.

Alle vorgenannten bevorzugten und besonders bevorzugten Mengenangaben gelten wieder unter der Maßgabe, dass das Gewichtsverhältnis G1/G2 innerhalb des erfindungsgemäßen Bereichs liegt.

Ein besonders bevorzugtes erfindungsgemäßes Produkt ist dadurch gekennzeichnet, dass

- die Zubereitung (a) das oder die verdickenden Polymere in einer Gesamtgewichtsmenge (G1) von 0,2 bis 9,5 Gew.-% - bezogen auf das Gesamtgewicht der Zubereitung (a) - enthält und
- die Zubereitung (b) das oder die verdickende Polymere in einer Gesamtgewichtsmenge (G2) von 0,2 bis 9,5 Gew.-% - bezogen auf das Gesamtgewicht der Zubereitung (b) - enthält.

Ein besonders bevorzugtes erfindungsgemäßes Produkt ist dadurch gekennzeichnet, dass

- die Zubereitung (a) das oder die verdickenden Polymere in einer Gesamtgewichtsmenge (G1) von 0,4 bis 7,5 Gew.-% - bezogen auf das Gesamtgewicht der Zubereitung (a) - enthält und
- die Zubereitung (b) das oder die verdickende Polymere in einer Gesamtgewichtsmenge (G2) von 0,4 bis 7,5 Gew.-% - bezogen auf das Gesamtgewicht der Zubereitung (b) - enthält.

Ein besonders bevorzugtes erfindungsgemäßes Produkt ist dadurch gekennzeichnet, dass

- die Zubereitung (a) das oder die verdickenden Polymere in einer Gesamtgewichtsmenge (G1) von 0,6 bis 5,5 Gew.-% - bezogen auf das Gesamtgewicht der Zubereitung (a) - enthält und
- die Zubereitung (b) das oder die verdickende Polymere in einer Gesamtgewichtsmenge (G2) von 0,6 bis 5,5 Gew.-

% - bezogen auf das Gesamtgewicht der Zubereitung (b) - enthält.

Ein besonders bevorzugtes erfindungsgemäßes Produkt ist dadurch gekennzeichnet, dass

- die Zubereitung (a) das oder die verdickenden Polymere in einer Gesamtgewichtsmenge (G1) von 0,8 bis 2,5 Gew.-% - bezogen auf das Gesamtgewicht der Zubereitung (a) - enthält und
- die Zubereitung (b) das oder die verdickende Polymere in einer Gesamtgewichtsmenge (G2) von 0,8 bis 2,5 Gew.-% - bezogen auf das Gesamtgewicht der Zubereitung (b) - enthält.

Der Einsatz von gleichen verdickenden Polymeren in aufeinander abgestimmten Gewichtsmengen in den Zubereitungen (a) und (b) gewährleistet die Entnahme von konstanten Mengenverhältnissen beider Formierungen über die Auslassöffnung (C), da auf diesem Wege die rheologischen Eigenschaften beider Formulierungen zuverlässig aufeinander abgestimmt werden können. Der Einsatz der speziellen Verdicker gewährleistet hierbei vor allem ein gleiches Fließverhalten bei ansonsten stark unterschiedlich zusammengesetzten Zubereitungen (a) und (b), das auch durch Unterschiede in der Spendergeometrie wenig beeinflusst wird. Hierbei hat sich die Einhaltung von speziellen Viskositätsbereichen als ganz besonders vorteilhaft erwiesen.

Ganz besonders bevorzugt ist daher ein Produkt zur oxidativen Farbänderung von keratinischen Fasern, welches dadurch gekennzeichnet ist, dass

- die Zubereitung (a) eine Viskosität von 1000 bis 100 000 mPas, bevorzugt von 2000 bis 80 000 mPas, weiter bevorzugt von 4000 bis 60 000 mPas, noch weiter bevorzugt von 7000 bis 50 000 mPas und besonders bevorzugt von 10 000 bis 40 000 mPas besitzt (22 °C / Brookfield-Viskosimeter / Spindel 5 / 4 Upm),
- die Zubereitung (b) eine Viskosität von 1000 bis 100 000 mPas, bevorzugt von 2000 bis 80 000 mPas, weiter bevorzugt von 4000 bis 60 000 mPas, noch weiter bevorzugt von 7000 bis 50 000 mPas und besonders bevorzugt von 10 000 bis 40 000 mPas besitzt (22 °C / Brookfield-Viskosimeter / Spindel 5 / 4 Upm) und
- das Verhältnis der Viskositäten V1/V2 bei einem Wert von 0,3 bis 3,0, bevorzugt von 0,4 bis 2,5, und besonders bevorzugt von 0,5 bis 2,0 liegt.

Bei den angegebenen Viskositäten handelt es sich um Viskositäten, die bei 22 °C gemessen wurden (22 °C / Brookfield-Viskosimeter / Spindel 5 / 4 Upm).

Noch besser lassen sich die rheologischen Eigenschaften der Zubereitungen (a) und (b) aufeinander abstimmen, wenn die beiden Zubeitungen zusätzlich auch einen ähnlichen bzw. einen gleichen Salzgehalt besitzen. In einer weiteren besonders bevorzugten Ausführungsform sind die Zubereitungen (a) und (b) daher zusätzlich durch ihren aufeinander abgestimmten Salzgehalt gekennzeichnet. Die Zubereitung (a) enthält bevorzugt ein oder mehrere organische und/oder anorganische Salze in einer Gesamtmolalität (M1) von 0,01 bis 1 mol/kg - bezogen auf das Gesamtgewicht der Zubereitung (a). Die Zubereitung (b) enthält weiterhin ein oder mehrere organische und/oder anorganische Salze in einer Gesamtmolalität (M2) von 0,01 bis 1 mol/kg - bezogen auf das Gesamtgewicht der Zubereitung (b). Erfindungswesentlich hierbei ist, dass das Molalitätsverhältnis M1/M2 bei einem Wert von 0,3 bis 3,0 liegt.

In einer weiteren ganz besonders bevorzugten Ausführungsform ist ein erfindungsgemäßes Produkt dadurch gekennzeichnet, dass

- die Zubereitung (a) zusätzlich ein oder mehrere organische und/oder anorganische Salze in einer Gesamtmolalität (M1) von 0,01 bis 1 mol/kg - bezogen auf das Gesamtgewicht der Zubereitung (a) enthält

- die Zubereitung (b) zusätzlich ein oder mehrere organische und/oder anorganische Salze in einer Gesamtmolalität (M2) von 0,01 bis 1 mol/kg - bezogen auf das Gesamtgewicht der Zubereitung (b) enthält und

- das Molalitätsverhältnis M1/M2 bei einem Wert von 0,3 bis 3,0 liegt.

Unter organischen und/oder anorganischen Salzen werden alle geladenen, d.h. ionischen Verbindungen von nicht polymerem Charakter verstanden. Die Verbindungen werden dann als nicht polymer eingestuft, wenn sie ein Molgewicht von maximal 5000 g/mol besitzen.

Polymere Verbindungen (d.h. Verbindungen mit einer molaren Masse von mehr als 5000 g/mol) fallen, auch wenn sie kationische und/oder anionische Ladungen tragen, nicht unter die Definition der organischen Salze. Der Grund hierfür ist, dass sie die rheologischen Eigenschaften der Zubereitung (a) und (b) nicht nur aufgrund ihrer Ladungen, sondern insbesondere aufgrund ihrer polymeren Struktur (insbesondere ihrer verdickenden Eigenschaften) beeinflussen. Bei der Berechnung der Gesamtmolalitäten M1 und M2 werden geladene polymere Verbindungen daher nicht berücksichtigt.

Beispiele für anorganische Salze sind Alkalimetallsulfate, Alkalimetallchloride, Alkalimetallbromide, Alkalimetallsulfate, Alkalimetallhydrogensulfate, Alkalimetallphophate, Alkalimetallhydroxide, Alkalimetallphosphate, Alkalimetallhydrogenphosphate, Erdalkalimetallsulfite, Erdalkalimetallchloride, Erdalkalimetallbromide, Erdalkalimetallsulfate, Erdalkalimetallhydrogensulfate, Erdalkalimetallphosphate, Erdalkalimetallhydroxide, Erdalkalimetallphosphate und Erdalkalimetallhydrogenphosphate.

Beispiele für organische Salze sind geladene Tenside, d.h. kationische Tenside, anionische Tenside und auch zwitterionische, in Form ihres inneren Salzes vorliegende Tenside.

Weitere Beispiele für organische Salze sind die in Form ihres Salzes vorliegenden Oxidationsfarbstoffvorprodukte. Oxidationsfarbstoffvorprodukte können in Entwickler und Kuppler unterteilt werden, wobei die Entwickler aufgrund ihrer größeren Empfindlichkeit gegenüber Sauerstoff meist in Form ihrer physiologisch verträglichen Salze (z.B. in Form ihrer Hydrochloride, Hydrobromide, Hydrogensulfate oder Sulfate) eingesetzt werden.

Kupplerkomponenten bilden im Rahmen der oxidativen Färbung allein keine signifikante Färbung aus, sondern benötigen stets die Gegenwart von Entwicklerkomponenten. Da Kuppler gegenüber Sauerstoff nicht so empfindlich sind wie Entwickler, können sie zwar ebenfalls in Form ihrer Salze in den Zubereitungen eingesetzt werden, werden oftmals aber auch in freier Form (d.h. nicht in Salzform) eingesetzt. Alle Entwickler und Kuppler, die in Form ihres Salzes in der Formulierung eingesetzt werden, fallen in die Gruppe der organischen Salze.

Bevorzugte Salze vom Entwickler-Typ sind p-Phenylendiamin x $H_2SO_4$, p-Phenylendiamin x 2 HCl, p-Toluylendiamin x $H_2SO_4$, p-Toluylendiamin x 2 HCl, 2-(2-Hydroxyethyl)-p-phenylendiamin x $H_2SO_4$, 2-(2-Hydroxyethyl)-p-phenylendiamin x 2 HCl, 2-(1,2-Dihydroxyethyl)-p-phenylendiamin x $H_2SO_4$, 2-(1,2-Dihydroxyethyl)-p-phenylendiamin x 2 HCl, N,N-Bis-(2-hydroxyethyl)-p-phenylendiamin x $H_2SO_4$, N,N-Bis-(2-hydroxyethyl)-p-phenylendiamin x 2 HCl, 2-Methoxymethyl-p-phenylendiamin x $H_2SO_4$, 2-Methoxymethyl-p-phenylendiamin x 2 HCl, N-(4-Amino-3-methylphenyl)-N-[3-(1H-imidazol-1-yl)propyl]amin x $H_2SO_4$, N-(4-Amino-3-methylphenyl)-N-[3-(1H-imidazol-1-yl)propyl]amin x 2 HCl, N-(4-Amino-3-methylphenyl)-N-[3-(1H-imidazol-1-yl)propyl]amin x 3 HCl, Bis-(2-hydroxy-5-aminophenyl)methan x $H_2SO_4$, Bis-(2-hydroxy-5-aminophenyl)methan x 2 HCl, 4,5-Diamino-1-(2-hydroxyethyl)-1H-pyrazol x $H_2SO_4$, 4,5-Diamino-1-(2-hydroxyethyl)-1H-pyrazol x 2 HCl, 2,4,5,6-Tetraaminopyrimidin x $H_2SO_4$, 2,4,5,6-Tetraaminopyrimidin x 2 H2SO4, 2,4,5,6-Tetraaminopyrimidin x 2 HCl, 2,4,5,6-Tetraaminopyrimidin x 3 HCl, 2,4,5,6-Tetraaminopyrimidin x 4 HCl, 4-Hydroxy-2,5,6-triaminopyrimidin x $H_2SO_4$, 4-Hydroxy-2,5,6-triaminopyrimidin x 2 HCl, 2-Hydroxy-4,5,6-triaminopyrimidin x $H_2SO_4$, 2-Hydroxy-4,5,6-triaminopyrimidin x 2 HCl, 2-Hydroxy-4,5,6-triaminopyrimidin x 3 HCl

Unter der Gesamtmolalität (M1) wird die Gesamtmolmenge aller in der Zubereitung (a) enthaltenen organischen und anorganischen Salze verstanden, wobei M1 auf das Gesamtgewicht der Zubereitung (a) bezogen ist (Einheit: mol Salz pro kg Formulierung (a)). In die Berechnung der Gesamtmolalität M1 werden alle organischen und anorganischen Salze mit einbezogen, die in Form ihres Salzes zu der Zubereitung (a) hinzugefügt werden.

Unter der Gesamtmolalität (M2) wird die Gesamtmolmenge aller in der Zubereitung (b) enthaltenen organischen und anorganischen Salze verstanden, wobei M2 auf das Gesamtgewicht der Zubereitung (b) bezogen ist (Einheit: mol Salz pro kg Formulierung (b)). In die Berechnung der Gesamtmolalität M2 werden alle organischen und anorganischen Salze mit einbezogen, die in Form ihres Salzes zu der Zubereitung (b) hinzugefügt werden.

Das Verhältnis der Molalitäten M1/M2 liegt bei einem Verhältnis von 0,3 bis 3,0. Hierbei ist das Mengenverhältnis der Formulierungen (a) und (b), das während der Anwendung aus dem Spender entnommen wird, umso konstanter und definierter, je ähnlicher die in beiden Formulierungen eingesetzten Salzmolalitäten sind, d.h. je näher das Molalitätsverhältnis M1/M2 bei einem Wert von 1 liegt.

In einer weiteren ganz besonders bevorzugten Ausführungsform ist ein Produkt zur oxidativen Färbung und/oder Aufhellung keratinischer Fasern daher dadurch gekennzeichnet, dass das Molalitätsverhältnis M1/M2 bei einem Wert von 0,4 bis 2,5, bevorzugt von 0,5 bis 2,0, weiter bevorzugt von 0,6 bis 1,7, und besonders bevorzugt von 0,7 bis 1,4 liegt.

Weiterhin besonders bevorzugt ist deshalb ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere der genannten verdickenden Polymere in einer Gesamtgewichtsmenge (G1) von 0,1 bis 40 Gew.-% - bezogen auf das Gesamtgewicht der Zubereitung (a),
- ein oder mehrere organische und/oder anorganische Salze in einer Gesamtmolalität (M1) von 0,01 bis 1 mol/kg - bezogen auf das Gesamtgewicht der Zubereitung (a),

(3) eine Zubereitung (b) in der Kammer (B), enthaltend in einem wässrigen, kosmetischen Träger

- ein oder mehrere der genannten verdickenden Polymere einer Gesamtgewichtsmenge (G2) von 0,1 bis 40 Gew.-% - bezogen auf das Gesamtgewicht der Zubereitung (b),
- ein oder mehrere organische und/oder anorganische Salze in einer Gesamtmolalität (M2) von 0,01 bis 1 mol/kg - bezogen auf das Gesamtgewicht der Zubereitung (b),
  dadurch gekennzeichnet, dass
- das Gewichtsverhältnis G1/G2 bei einem Wert von 0,5 bis 2,0 liegt und
- das Molalitätsverhältnis M1/M2 bei einem Wert von 0,3 bis 3,0 liegt, bevorzugt von 0,4 bis 2,5, weiter bevorzugt von 0,5 bis 2,0, noch weiter bevorzugt von 0,6 bis 1,7, und bevorzugt von 0,7 bis 1,4 liegt.

Weiterhin besonders bevorzugt ist deshalb ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere der genannten verdickenden Polymere in einer Gesamtgewichtsmenge (G1) von 0,1 bis 40 Gew.-% - bezogen auf das Gesamtgewicht der Zubereitung (a),
- ein oder mehrere organische und/oder anorganische Salze in einer Gesamtmolalität (M1) von 0,01 bis 1 mol/kg - bezogen auf das Gesamtgewicht der Zubereitung (a),

(3) eine Zubereitung (b) in der Kammer (B), enthaltend in einem wässrigen, kosmetischen Träger

- ein oder mehrere der genannten verdickenden Polymere einer Gesamtgewichtsmenge (G2) von 0,1 bis 40 Gew.-% - bezogen auf das Gesamtgewicht der Zubereitung (b),
- ein oder mehrere organische und/oder anorganische Salze in einer Gesamtmolalität (M2) von 0,01 bis 1 mol/kg - bezogen auf das Gesamtgewicht der Zubereitung (b),
  dadurch gekennzeichnet, dass
- das Gewichtsverhältnis G1/G2 bei einem Wert von 0,55 bis 1,8 liegt und
- das Molalitätsverhältnis M1/M2 bei einem Wert von 0,3 bis 3,0 liegt, bevorzugt von 0,4 bis 2,5, weiter bevorzugt von 0,5 bis 2,0, noch weiter bevorzugt von 0,6 bis 1,7, und bevorzugt von 0,7 bis 1,4 liegt.

Weiterhin besonders bevorzugt ist deshalb ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere der genannten verdickenden Polymere in einer Gesamtgewichtsmenge (G1) von 0,1 bis 40 Gew.-% - bezogen auf das Gesamtgewicht der Zubereitung (a),
- ein oder mehrere organische und/oder anorganische Salze in einer Gesamtmolalität (M1) von 0,01 bis 1 mol/kg - bezogen auf das Gesamtgewicht der Zubereitung (a),

(3) eine Zubereitung (b) in der Kammer (B), enthaltend in einem wässrigen, kosmetischen Träger

- ein oder mehrere der genannten verdickenden Polymere einer Gesamtgewichtsmenge (G2) von 0,1 bis 40 Gew.-% - bezogen auf das Gesamtgewicht der Zubereitung (b),
- ein oder mehrere organische und/oder anorganische Salze in einer Gesamtmolalität (M2) von 0,01 bis 1 mol/kg - bezogen auf das Gesamtgewicht der Zubereitung (b),

dadurch gekennzeichnet, dass

- das Gewichtsverhältnis G1/G2 bei einem Wert von 0,6 bis 1,6 liegt und
- das Molalitätsverhältnis M1/M2 bei einem Wert von 0,3 bis 3,0 liegt, bevorzugt von 0,4 bis 2,5, weiter bevorzugt von 0,5 bis 2,0, noch weiter bevorzugt von 0,6 bis 1,7, und bevorzugt von 0,7 bis 1,4 liegt.

Weiterhin besonders bevorzugt ist deshalb ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere der genannten verdickenden Polymere in einer Gesamtgewichtsmenge (G1) von 0,1 bis 40 Gew.-% - bezogen auf das Gesamtgewicht der Zubereitung (a),
- ein oder mehrere organische und/oder anorganische Salze in einer Gesamtmolalität (M1) von 0,01 bis 1 mol/kg - bezogen auf das Gesamtgewicht der Zubereitung (a),

(3) eine Zubereitung (b) in der Kammer (B), enthaltend in einem wässrigen, kosmetischen Träger

- ein oder mehrere der genannten verdickenden Polymere einer Gesamtgewichtsmenge (G2) von 0,1 bis 40 Gew.-% - bezogen auf das Gesamtgewicht der Zubereitung (b),
- ein oder mehrere organische und/oder anorganische Salze in einer Gesamtmolalität (M2) von 0,01 bis 1 mol/kg - bezogen auf das Gesamtgewicht der Zubereitung (b),
  dadurch gekennzeichnet, dass
- das Gewichtsverhältnis G1/G2 bei einem Wert von 0,7 bis 1,4 liegt und
- das Molalitätsverhältnis M1/M2 bei einem Wert von 0,3 bis 3,0 liegt, bevorzugt von 0,4 bis 2,5, weiter bevorzugt von 0,5 bis 2,0, noch weiter bevorzugt von 0,6 bis 1,7, und bevorzugt von 0,7 bis 1,4 liegt.

Aufgrund der unterschiedlichen Funktionen der Zubereitungen (a) und (b) enthalten beide Zubereitungen definitionsgemäß unterschiedliche Salze.

Ein besonders bevorzugtes Produkt ist dadurch gekennzeichnet, dass die Zubereitung (a) ein oder mehrere organische und/oder anorganische Salze aus der Gruppe der salzartigen Oxidationsfarbstoffvorprodukte, der Alkalimetallsalze der 1-Hydroxyethan-1,1-diphosphonsäure, der Alkalimetallsulfite, der Alkalimetallchloride, der Alkalimetallbromide, der Erdalkalimetallchloride, der Erdalkalimetallbromide, der Alkalimetallsulfate, der Erdalkalimetallsulfate, der Alkalimetallhydrogensulfate, der Erdalkalimetallhydrogensulfate, der Alkalimetallphosphate und/oder der Erdalkalimetallphosphate enthält.

In einer weiteren besonders bevorzugten Ausführungsform ist ein Produkt zur oxidativen Farbänderung dadurch gekennzeichnet, dass die Zubereitung (b) ein oder mehrere organische und/oder anorganische Salze aus der Gruppe der Alkalimetallsalze der 1-Hydroxyethan-1,1-diphosphonsäure, der Alkalimetallsalze der Dipicolinsäure, der Alkalimetallhydroxide, der Alkalimetallbenzoate, der Alkalimetallchloride, der Erdalkalimetallchloride, der Alkalimetallbromide, der Erdalkalimetallbromide, der Alkalimetallcarbonate, der der Erdalkalimetallhydrogencarbonate, der Alkalimetallsulfate, der Erdalkalimetallsulfate, der Alkalimetallhydrogensulfate, der Erdalkalimetallhydrogensulfate, der Alkalimetallphosphate, der Erdalkalimetallphosphate, der Alkalimetallpyrophosphate und/oder der Erdalkalimetallpyrophosphate enthält.

Die Gesamtmolalitäten der in den Zubereitungen (a) und (b) enthaltenen Salze werden hierbei jedoch wie zuvor beschrieben aufeinander abgestimmt, was bedeutet, dass Molalitätsverhältnis M1/M2 im erfindungsgemäßen bzw. im bevorzugten oder besonders bevorzugten Wertebereich liegt. Die beste Dosierbarkeit wird bei möglichst ähnlichen Salzgehalten erreicht.

In den Formulierungen (a) und (b) können hierbei sowohl Salze aus einfach positiv geladen Kationen mit einfach negativ geladenen Anionen (z.B. Natriumchlorid), wie auch die Salze von mehrfach positiv geladenen Kationen mit einfach negativ geladenen Anionen (z.B. Magnesiumchlorid), wie auch die Salze von einfach positiv geladenen Kationen mit mehrfach negativ geladenen Anionen (z.B. Natriumsulfat), wie schließlich auch die Salze von mehrfach positiv geladenen Kationen mit mehrfach negativ geladenen Anionen (z.B. Magnesiumsulfat) - jeweils unter Einhaltung der für die Gesamtneutralität des Salzes notwendigen stöchiometrischen Voraussetzungen - eingesetzt werden.

In diesem Zusammenhang konnte beobachtet werden, dass nicht nur die Konzentration (d.h. Molalität) der in

den Zubereitungen (a) und (b) enthaltenen Salze einen Einfluss auf die Dosierbarkeit der Zubereitungen (a) und (b) ausübt, sondern dass darüber hinaus auch Salze mit möglichst gleicher Ladungszahl in beiden Formulierungen (a) und (b) eingesetzt werden sollten.

Mit anderen Worten konnten dem Produkt besonders dann Anwendungsmischungen mit reproduzierbarer Zusammensetzung entnommen werden, wenn die Formulierungen (a) und (b) die organischen/anorganischen Salze nicht nur in ähnlichen Mengen enthielten, sondern wenn auch die Ladungszahl der in (a) und (b) eingesetzten Salze möglichst ähnlich war. Die Ladungszahl der eingesetzten Salze lässt sich über die Ionenstärke quantifizieren. Die Ionenstärke ist ein ladungsabhängiges Konzentrationsmaß für elektrolytische Lösungen.

In einer weiteren besonders bevorzugten Ausführungsform ist ein Produkt zur oxidativen Farbveränderung von keratinischen Fasern dadurch gekennzeichnet, dass

- die Zubereitung (a) ein oder mehrere organische und/oder anorganische Salze in einer Ionenstärke (I1) enthält, die sich nach der Formel (1) berechnet

$$I1 = \frac{1}{2}\sum_i z_i^2 c_i \qquad (1)$$

  mit zi gleich der Ladungszahl jedes in der Zubereitung (a) enthaltenen Ions (i) und
  ci gleich der Molalität (mol/kg) jedes in der Zubereitung (a) enthaltenen Ions, wobei sich die Molalität auf das Gesamtgewicht der Zubereitung (a) bezieht,
- die Zubereitung (b) ein oder mehrere organische und/oder anorganische Salze in einer Ionenstärke (I2) enthält, die sich nach der Formel (2) berechnet

$$I2 = \frac{1}{2}\sum_i z_i^2 c_i \qquad (2)$$

  mit zi gleich der Ladungszahl jedes in der Zubereitung (b) enthaltenen Ions (i) und
  ci gleich der Molalität (mol/kg) jedes in der Zubereitung (b) enthaltenen Ions, wobei sich die Molalität auf das Gesamtgewicht der Zubereitung (b) bezieht, und
- das Verhältnis der Ionenstärken I1/I2 bei einem Wert von 0,5 bis 2,0, bevorzugt von 0,6 bis 1,8, weiter bevorzugt von 0,7 bis 1,6 liegt.

Die Ionenstärken I1 und I2 gemäß obiger Definition berücksichtigen die Ladungen und Molalitäten jedes einzelnen in den Zubereitungen (a) und (b) enthaltenen Ions.

Berechnungsgrundlage für die Molalität ci der Ionenstärke I1 ist das Gesamtgewicht der Zubereitung (a). Berechnungsgrundlage für die Molalität ci der Ionenstärke I2 ist das Gesamtgewicht der Zubereitung (b).

Beispiel:

Zubereitung (a) enthält 0,2 Gew.-% p-Toluylendiamin, Sulfatsalz ($C_7H_{12}N_2^{2+}$ x $SO_4^{2-}$) (molare Masse = 220,25 g/mol);

1000 g der Formulierung (a) enthalten 2 g p-Toluylendiamin, Sulfatsalz = 9,08 x $10^{-3}$ mol Molalität = 9,08 x $10^{-3}$ mol/kg, Ladungszahl Kation = 2, Ladungszahl Anion = 2

I1 = 2 x (9,08 x $10^{-3}$) mol/kg ($C_7H_{12}N_2^{2+}$) + 2 x (9,08 x $10^{-3}$) mol/kg ($SO_4^{2-}$) = 0,03632

Zubereitung (b) enthält 0,11 Gew.-% Magnesiumsulfat ($MgSO_4$) (molare Masse = 120,37 g/mol) 1000 g der Formulierung (b) enthalten 1,1 g Magnesiumsulfat = 9,14 x $10^{-3}$ mol Molalität = 9,14 x $10^{-3}$ mol/kg, Ladungszahl Kation = 2, Ladungszahl Anion = 2

I2 = 2 x (9,14 x $10^{-3}$ mol/kg) + 2 x 9,14 x $10^{-3}$ mol/kg = 0,03655

I1/I2 = 0,994

Die Zubereitung (a) kann zusätzlich auch noch ein oder mehrere Oxidationsfarbstoffvorprodukte enthalten, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Alle Oxidationsfarbstoffvorprodukte, die in ihrer Salzform eingesetzt werden, müssen hierbei bei der Berechnung der Gesamtmolalität M1 berücksichtigt werden.

Zusätzlich kann die Zubereitung (a) ebenfalls mindestens einen direktziehenden Farbstoff aus der Gruppe der anionischen, nichtionischen und/oder kationischen Farbstoffe enthalten. Die anionischen und kationischen direktziehenden Farbstoffe fallen unter die Gruppe der organischen Salze und müssen ebenfalls bei der Berechnung der Gesamtmolalität M1 berücksichtigt werden.

Die Oxidationsfarbstoffvorprodukte, d.h. Entwicklerkomponenten und Kupplerkomponenten, sowie die optional zusätzlich enthaltenen direktziehenden Farbstoffe können beispielsweise in einer Menge von 0,0001 bis 5,0 Gew.-%, vorzugsweise 0,001 bis 3,5 Gew.-%, jeweils bezogen auf das Gesamtgewicht der Zubereitung (a) verwendet werden.

Den Zubereitungen (a) und (b) wird bevorzugt zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Zubereitungen (a) und (b) sind dadurch gekennzeichnet, daß sie zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt. Erfindungsgemäß bevorzugte Zubereitungen (a) und (b) sind dadurch gekennzeichnet, daß die Zubereitungen (a) und/oder (b) zusätzlich mindestens ein zwitterionisches Tensid enthalten. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethylimidazoline. Ein bevorzugtes zwitterionisches Tensid ist unter der INCI-Bezeichnung Cocamidopropyl Betaine bekannt.

Erfindungsgemäß bevorzugte Zubereitungen (a) und (b) sind dadurch gekennzeichnet, daß die Zubereitungen (a) und/oder (b) zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und $C_{12}$-$C_{18}$-Acylsarcosin.

Die nichtionischen, zwitterionischen oder amphoteren Tenside können in Anteilen von 0,1 bis 40 Gew.%, bevorzugt 1 bis 30 Gew.% und ganz besonders bevorzugt von 1 bis 15 Gew.%, jeweils bezogen auf die Gesamtmenge der Zubereitungen (a) und (b), eingesetzt werden.

Alle in den Zubereitungen (a) und (b) enthaltenen anionischen, kationischen und zwitterionischen Tenside fallen in die Gruppe der organischen Salze, ihre Einsatzkonzentrationen müssen daher bei der Berechnung der Gesamtmolalitäten M1 bzw. M2 berücksichtigt werden.

Weiterhin hat es sich als vorteilhaft erwiesen, wenn die Mittel weitere, nichtionogene grenzflächenaktive Stoffe, enthalten. Bevorzugte nichtionische Tenside sind beispielsweise Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

In einer weiteren ganz besonders bevorzugten Ausführungsform enthalten die Zubereitungen (a) und (b) auch jeweils ähnliche bzw. gleiche Mengen an nichtionischen Tensiden

Zusätzlich können die Zubereitungen (a) und (b) auch einen oder mehrere Fettbestandteile enthalten. Unter Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden.

Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 8 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Geeignete Fettbestandteile sind beispielsweise $C_{12}$-$C_{30}$-Fettalkohole, $C_{12}$-$C_{30}$-Fettsäuretriglyceride, $C_{12}$-$C_{30}$-Fettsäuremonoglyceride, $C_{12}$-$C_{30}$-Fettsäurediglyceride und/oder Kohlenwasserstoffe. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{30}$-Fettalkoholen kann es sich um gesättigte, ein- oder mehrfach ungesättigte, lineare oder verzweigte Fettalkohole mit 12 bis 30 C-Atomen handeln.

Beispiele für geeignete lineare, gesättigte $C_{12}$-$C_{30}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Geeignete lineare, ungesättigte Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol) und/oder Brassidylalkohol ((13*E*)-Docosen-1-ol).

Unter einem $C_{12}$-$C_{30}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{30}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Beispielhaft für Fettsäure- Mono-, Di und Triglyceride können Triglyceride genannt werden, bei welchen mindestens eine der Estergruppen ausgehend von Glycerin mit einer Fettsäure ausgebildet wird, die ausgewählt wird aus Docdecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9*Z*)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{30}$-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein. Unter einem $C_{12}$-$C_{30}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als geeignet haben sich in diesem Zusammenhang flüssige Paraffinöle (Paraffinum Liquidum und Paraffinium Perliquidum) erwiesen. Bei dem Kohlenwasserstoff kann es sich beispielsweise um Paraffinum Liquidum, auch Weißöl

genannt, handeln. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Auch wenn die Zubereitungen (a) und (b) Fettbestandteile enthalten können, so sind sie in einer besonders bevorzugten Ausführungsform doch frei von Fettbestandteilen.

Unter der Bezeichnung "frei von Fettbestandteilen" wird im Sinn der vorliegenden Erfindung verstanden, dass die Zubereitung (a) eine Gesamtgewichtsmenge an Fettbestandteilen von weniger als 2,5, Gew.-% bevorzugt von weniger als 1,0 Gew.-%, besonders bevorzugt weniger als 0,5 Gew.-% - bezogen auf das Gesamtgewicht der Zubereitung (a) - enthält.

Unter der Bezeichnung "frei von Fettbestandteilen" wird im Sinn der vorliegenden Erfindung verstanden, dass die Zubereitung (b) eine Gesamtgewichtsmenge an Fettbestandteilen von weniger als 2,5, Gew.-% bevorzugt von weniger als 1,0 Gew.-%, besonders bevorzugt weniger als 0,5 Gew.-% - bezogen auf das Gesamtgewicht der Zubereitung (b) - enthält.

Weiterhin besonders bevorzugt ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- einen oder mehrere der genannten verdickenden Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- einen oder mehrere der genannten verdickenden Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0 liegt und
- die Gesamtmenge an Fettbestandteilen in der Zubereitung (a) weniger als 2,5 Gew.-%, bevorzugt weniger als 1,0 Gew.-%, besonders bevorzugt weniger als 0,5 Gew.-% - bezogen auf das Gesamtgewicht der Zubereitung (a) - beträgt, und
- die Gesamtmenge an Fettbestandteilen in der Zubereitung (b) weniger als 2,5 Gew.-%, bevorzugt weniger als 1,0 Gew.-%, besonders bevorzugt weniger als 0,5 Gew.-% - bezogen auf das Gesamtgewicht der Zubereitung (b) - beträgt.

Bei der Zubereitung (a) handelt es sich bevorzugt eine alkalisch eingestellte Formulierung, die gegebenenfalls - wenn zusätzlich zur Aufhellung auch eine Farbveränderung gewünscht ist, die Oxidationsfarbstoffvorprodukte enthält. Bei der Zubereitung (b) handelt es sich um eine - aus Stabilitätsgründen of sauer eingestellte - Formulierung mit Oxidationsmittel.

In einer weiteren besonders bevorzugten Ausführungsform ist ein erfindungsgemäßes Produkt daher dadurch gekennzeichnet, dass

- die Zubereitung (a) mindestens ein Alkalisierungsmittel, bevorzugt Ammoniak und/oder Monoethanolamin, und
- die Zubereitung (b) mindestens ein Oxidationsmittel, bevorzugt Wasserstoffperoxid, enthält.

Die Zubereitung (a) enthält bevorzugt mindestens ein Alkalisierungsmittel, bevorzugt Ammoniak und/oder Monoethanolamin. Erfindungsgemäß verwendbare Alkalisierungsmittel können aus der Gruppe ausgewählt sein, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel können ausgewählt sein aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

Üblicherweise wird Ammoniak ($NH_3$) in Form seiner wässrigen Lösung eingesetzt. Wässrige Ammoniak-Lösungen enthalten Ammoniak ($NH_3$) oft in Konzentrationen zwischen 10 Gew.-% und 32 Gew.-%. Bevorzugt ist hierbei der Einsatz einer wässrigen Ammoniak-Lösung, die 25 Gew.-% Ammoniak ($NH_3$) enthält.

Bevorzugt sind Ammoniak und/oder Monoethanolamin in der Zubereitung (a) in Mengen von 0,01 bis 10 Gew.-%, bevorzugt von 0,1 bis 7,5 Gew.-%, weiter bevorzugt von 0,2 bis 5,5 Gew.-% und besonders bevorzugt von 0,4 bis 4,5 Gew.-% - jeweils bezogen auf das Gesamtgewicht der Zubereitung (a) - enthalten.

Werden die Alkalisierungsmittel in Salzform, wie beispielsweise Kaliumhydroxid oder Natriumhydroxid, eingesetzt, so müssen deren Einsatzkonzentrationen auch bei der Berechnung der Gesamtmolalität M1 (sowie M2) berücksichtigt werden.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels (d.h. der Mischung aus den Zubereitungen (a) und (b)) zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Hierbei ist die Zubereitung (a) bevorzugt auf einen alkalischen pH-Wert eingestellt, und die Zubereitung (b) besitzt in vorteilhafter Weise einen sauren pH-Wert.

In einer weiteren besonders bevorzugten Ausführungsform ist ein erfindungsgemäßes Produkt dadurch gekennzeichnet, dass

- die Zubereitung (a) einen pH-Wert von 8 bis 12,5, bevorzugt von 8,5 bis 12, weiter bevorzugt von 9 bis 11,5 und besonders bevorzugt von 9,5 bis 11 besitzt
- die Zubereitung (b) einen pH-Wert von 1 bis 7, bevorzugt von 1,5 bis 6, weiter bevorzugt von 2,5 bis 5,5 und besonders bevorzugt von 3,0 bis 5,0 besitzt.

In einer weiteren besonders bevorzugten Ausführungsform ist ein erfindungsgemäßes Produkt dadurch gekennzeichnet, dass

- die Zubereitung (a) einen pH-Wert von 8,5 bis 12 besitzt und
- die Zubereitung (b) einen pH-Wert von 1,5 bis 6 besitzt.

In einer weiteren besonders bevorzugten Ausführungsform ist ein erfindungsgemäßes Produkt dadurch gekennzeichnet, dass

- die Zubereitung (a) einen pH-Wert von 9 bis 11,5 besitzt und
- die Zubereitung (b) einen pH-Wert von 2,5 bis 5,5 besitzt.

In einer weiteren besonders bevorzugten Ausführungsform ist ein erfindungsgemäßes Produkt dadurch gekennzeichnet, dass

- die Zubereitung (a) einen pH-Wert von 9,5 bis 11 besitzt und
- die Zubereitung (b) einen pH-Wert von 3,0 bis 5,0 besitzt.

Wasserstoffperoxid wird bevorzugt als wässrige Lösung in der Zubereitung (b) verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (b) sind dadurch gekennzeichnet, dass sie, bezogen auf das Gesamtgewicht der Zubereitung (b), 0,5 bis 20 Gew.-%, vorzugsweise 1 bis 12,5 Gew.-% Wasserstoffperoxid, jeweils bezogen auf das Gesamtgewicht des Mittels, enthalten.

Die Zubereitungen (a) und (b) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Es hat sich als vorteilhaft erweisen, wenn die Färbemittel (d.h. die Zubereitungen (a) und insbesondere (b)) insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat), Salicylsäure und Dipicolinsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Alle in den Zubereitungen (a) und (b) enthaltenen ionischen Komplexbildner fallen in die Gruppe der organischen

Salze, ihre Einsatzkonzentrationen müssen daher bei der Berechnung der Gesamtmolalitäten M1 bzw. M2 berücksichtigt werden.

Ferner können die erfindungsgemäßen Zubereitungen weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Zubereitungen (a) und (b) treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Unter dem Spender wird im Sinne der vorliegenden Erfindung ein zumindest zwei Kammern ((A) und (B)) umfassendes Behältnis verstanden, das beispielsweise aus Kunststoff, Metall und/oder Kunststoff-Metall-Verbundstoffen gefertigt sein kann. Der Spender kann u. a. in Form einer Dose, einer Flasche, eines Containers, eines Druckbehälters oder einer Tube vorliegen. Für das erfindungsgemäße Produkt sind alle Ausgestaltungen des Spenders geeignet, die gewährleisten, dass die in den beiden Kammern des Spenders vorliegenden Produktzusammensetzungen (a) und (b) bis zur Entnahme zuverlässig voneinander isoliert gehalten werden.

Vorzugsweise werden die einzelnen Produktzusammensetzungen im Zuge der Produktanwendungen zu einer Gesamtformulierung vermischt, um auf diesem Wege die vollständige Wirksamkeit der Gesamtformulierung auszuschöpfen sowie die Produktanwendung allgemein zu erleichtern. Zu diesem Zweck umfasst der Spender sinnvollerweise neben den Kammern (A), (B) einen Ausgabekopf, innerhalb dessen die Produktzusammensetzungen (a), (b) von den Kammern zur Auslassöffnung (C) befördert werden. Dabei ist im Ausgabekopf eine geeignete Mischvorrichtung ausgebildet, welche stromaufwärts der Auslassöffnung (C) für die gewünschte Durchmischung der Produktzusammensetzungen (a), (b) sorgt, bevor die durchmischte Gesamtformulierung, d. h. (a) + (b) über die Auslassöffnung (C) abgegeben wird. Beispielsweise aus der DE 3729491 A1 ist ein gattungsgemäßer Spender mit einer vergleichbaren Mischvorrichtung bekannt, wobei die dortige Mischvorrichtung jedoch nur eine sehr kurze Mischstrecke aufweist. Erfindungsgemäß ist eine derartige Mischvorrichtung unmittelbar in den Ausgabekopf baulich integriert oder aber alternativ als separates Bauteil innerhalb des Ausgabekopfes angeordnet. Als geeignete Mischvorrichtungen im Sinne der Erfindung sind beispielsweise Statikmischer oder vergleichbar wirkende Mischstrecken zu verstehen, die von den fließfähigen Produktzusammensetzungen (a), (b) durchströmt werden und im Zuge dieser Durchströmung hinreichend vermischt werden. Dazu weist eine solche Mischstrecke üblicherweise geeignete Strömungseinbauten oder Strömungsstörer auf, die bei Umströmung aufgrund von erzeugten Turbulenzen eine Durchmischung einzelner Fluidkomponenten bewirken. Entscheidend für die Güte der Durchmischung einzelner Fluidkomponenten, hier der Produktzusammensetzungen (a), (b), innerhalb der Mischvorrichtung ist u. a. die zielgerichtete Abstimmung der Länge der Mischstrecke sowie die Gestaltung der Strömungseinbauten auf die rheologischen Eigenschaften der Produktzusammensetzungen (a), (b). Üblicherweise sind für hinreichende Mischergebnisse der erfindungsgemäßen Mischvorrichtung definierte Mindestlängen der Mischstrecke erforderlich. Daher ist die Mischstrecke der erfindungsgemäßen Mischvorrichtung bevorzugt derart gestaltet, dass zwar eine definierte Mindestlänge der Mischstrecke garantiert ist, ohne eine kompakte Gesamtkonstruktion der Mischvorrichtung und somit des Ausgabekopfes aufzugeben. So werden ausreichende Mischergebnisse gewährleistet und gleichzeitig gewünscht kompakte Außenabmessungen der Mischvorrichtung und des Ausgabekopfes sichergestellt. Dazu ist die Mischstrecke beispielsweise spiralförmig oder vergleichbar kompakt innerhalb des Ausgabekopfes ausgebildet.

Die Kammer (B) des Spenders kann neben, über oder unter der Kammer (A) angeordnet sein. Weiterhin kann die Kammer (B) innerhalb der Kammer (A) oder die Kammer (A) innerhalb der Kammer (B) liegen, wobei jeweils die eine Kammer die andere teilweise oder vollständig umhüllt. Das Fassungsvermögen der Kammern (A) und (B) kann jeweils 10 $cm^3$ bis 1000 $cm^3$ betragen, und das Fassungsvermögen der Kammern (A) und (B) kann gleich oder unterschiedlich sein. Bevorzugt ist das Fassungsvermögen der Kammern (A) und (B) gleich. Bevorzugt sind die Kammern A und B nebeneinander angeordnet.

Der Spender besitzt eine Auslassöffnung (C), die mit den beiden Kammern (A) und (B) in Fluidverbindung steht bzw. über Ventile mit diesem in Fluidverbindung gebracht werden kann. Während der Entnahme Anwendungszusammensetzung werden gleichzeitig die beiden Formulierungen (a) und (b) über die gemeinsame Auslassöffnung entnommen und vorzugsweise bereits stromauf mittels der oben beschriebenen Mischvorrichtung miteinander vermischt. Damit kommen die üblicherweise reaktiven Einzelformulierungen (a) und (b) erst während der Produktanwendung miteinander in Kontakt und bilden hierbei die anwendungsbereite Mischung.

Bei der Auslassöffnung (C) kann es sich zum Beispiel um eine Druckbehälter-Abgabevorrichtung, ein Pumpelement, ein Ventil oder eine Abgabevorrichtung für pastöse Massen handeln. Die Entnahme der Zubereitungen (a) und (b) über die gemeinsame Auslassöffnung (C) kann durch Drücken des Ventils oder durch Pumpen des Pumpelements erfolgen.

Bei dem Spender kann es sich auch um einen Quetschbehälter handeln, in diesem Fall erfolgt die Entnahme der Zubereitungen (a) und (b) bevorzugt durch Drücken des Quetschbehälters, wobei der Druck auf den Quetschbehälter zum Austritt der Zubereitungen (a) und (b) aus der gemeinsamen Auslassöffnung (C) führt.

Weiterhin sind auch alle Ausgestaltungen einer Auslassöffnung (C) denkbar, die es ermöglichen, dass die Formulierungen (a) und (b) gemeinsam aus dem Spender austreten.

Ein besonders bevorzugtes Produkt ist dadurch gekennzeichnet, dass

- es sich bei dem Spender um einen Druckbehälter handelt
- es sich bei der Auslassöffnung (C) um eine Druckbehälter-Abgabevorrichtung handelt, die auch eine entsprechende Mischvorrichtung aufweist, und dass
- der Spender zusätzlich mindestens ein Treibgas aus der Gruppe Propan, Propen, n-Butan, iso-Butan, iso-Buten, n-Pentan, Penten, iso-Pentan, iso-Penten, Luft, Stickstoff, Argon, $N_2O$ und/oder $CO_2$ enthält.

In der vorgenannten bevorzugten Ausführungsform umfasst das erfindungsgemäße Produkt einen Druckbehälter. Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke können bei Bedarf die Korrosionsbeständigkeit gegenüber der im Druckbehälter enthaltenen Zubereitung gewährleisten.

Ganz besonders bevorzugt handelt es sich bei den beiden voneinander getrennten Kammern (A) und (B) um zwei verformbare Beutel, vorzugsweise aus laminiertem Aluminium, welche jeweils mit der Auslassöffnung (C) in Verbindung stehen. Bei der Auslassöffnung (C) handelt es sich um eine Druckbehälter-Abgabevorrichtung. Beide Beutel befinden sich in einem dosenförmigen Druckbehälter, wobei der Druckbehälter zusammen mit der Druckbehälterabgabevorrichtung den Spender nach außen druckdicht abschließt. Der Raum zwischen der Außenwandung der Beutel und der Innenwandung des Druckbehälters ist mit mindestens einem Treibgas befüllt. Entsprechende Spender sind beispielsweise aus US 2009/0108021 A1 bekannt. Ein ganz besonders bevorzugtes Produkt zur Behandlung keratinischer Fasern zeichnet sich daher durch einen solchen Spender mit entsprechendem Druckbehälter aus, welcher auch die oben beschriebene Mischvorrichtung aufweist. Eine derartige ausgebildeter Spender trägt nicht nur der chemischen Reaktivität der verwendeten Produkteinzelzusammensetzungen (a), (b) Rechnung, sondern sorgt darüber hinaus als Folge der Durchmischung auch für eine hohe Wirksamkeit der angewendeten Gesamtformulierung. Besonders gute erfindungsgemäße Effekte werden erzielt, wenn der Innendruck des Druckbehälters mindestens 1,8 bar, insbesondere mindestens 2,5 bar, beträgt. Das Produkt umfasst weiterhin eine Abgabevorrichtung (entsprechend der Auslassöffnung C), welche zur Abgabe des Anwendungsmischung ein Ventil besitzen kann. In einer bevorzugten Ausführungsform der Erfindung weist das Ventil einen mit einem Lack oder einem polymeren kunststoffbeschichteten Ventilteller und ein ebensolches flexibles Element mit Rückstellcharakteristik auf, dass das Ventil nach Beenden der Betätigung in die Verschlussstellung (= Ruhelage des Ventils) zurückstellt. Entsprechende kosmetische Produkte, bei denen die Aerosol-Abgabevorrichtung ein Ventil umfasst, das einen Ventilkegel und/oder ein flexibles Element mit Rückstellcharakteristik aufweist, der/das/die mit einem Lack oder einem polymeren Kunststoff beschichtet ist/sind, sind erfindungsgemäß ebenfalls bevorzugt.

In einer weiteren bevorzugten Ausführungsform der Erfindung weist das Ventil ein flexibles Element mit Rückstellcharakteristik und/oder einen Ventilteller aus mindestens einem Kunststoff, bevorzugt einem elastomeren Kunststoff, auf. Auch hier sind erfindungsgemäße kosmetische Produkte, bei denen das Ventil ein flexibles Element mit Rückstellcharakteristik und/oder einen Ventilkegel aus mindestens einem Kunststoff aufweist, bevorzugt, wobei bevorzugte Kunststoffe elastomere Kunststoffe sind. Besonders bevorzugte elastomere Kunststoffe sind ausgewählt aus Buna, insbesondere Buna N, Buna 421, Buna 1602 und Buna KA 6712, Neopren, Butyl und Chlorbutyl.

In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element des Ventils mit Rückstellcharakteristik einstückig mit dem Ventilkegel ausgebildet sein und biegsame Beine aufweisen. Eine solche Feder kann aus Metall oder Kunststoff sein.

Alle erfindungsgemäß verwendeten Ventile weisen vorzugsweise einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten Wasser in Öl Emulsionen ebenfalls innen lackiert oder beschichtet sein.

Wenn das erfindungsgemäße Produkt über einen Druckbehälter appliziert wird, enthalten die Spender zusätzlich mindestens ein Treibgas aus der Gruppe Propan, Propen, n-Butan, iso-Butan, iso-Buten, n-Pentan, Penten, iso-Pentan, iso-Penten, Luft, Stickstoff, Argon, $N_2O$ und/oder $CO_2$. Innerhalb dieser Gruppe sind die permanenten Gase Luft, Stickstoff, Argon, $N_2O$ und/oder $CO_2$ bevorzugt, ganz besonders bevorzugt sind Stickstoff, Argon und/oder $CO_2$.

Weiterhin hat es sich als bevorzugt herausgestellt, wenn auch die Treibgase in bestimmten Drücken im Spender enthalten sind. In einer bevorzugten Ausführungsform enthalten die erfindungsgemäßen Spender ein oder mehrere weitere Treibgase daher mit einem Druck von 3 - 12 bar, bevorzugt von 4 bis 10 bar, und besonders bevorzugt von 5 bis 8 bar-jeweils bezogen auf den Druck der Treibgase zwischen den beutelförmigen Kammern (A) und (B) und dem Druckbehälter.

In einer weiteren besonders bevorzugten Ausführungsform handelt es sich bei dem Spender um einen Spender für pastöse Massen, eine Mehrkammertube oder einen Quetschbehälter.

In einer weiteren ganz besonders bevorzugten Ausführungsform ist ein erfindungsgemäßes Produkt dadurchgekennzeichnet, dass

- es sich bei dem Spender um einen Spender für pastöse Massen, eine Tube oder einen Quetschbehälter handelt und
- es sich bei der Auslassöffnung (C) um ein Pumpelement, ein Ventil oder eine Abgabevorrichtung für pastöse Massen handelt.

Das erfindungsgemäße Produkt umfasst gemäß einer alternativen Gestaltungsform eine Mehrkammertube, die eine innere und eine äußere Kammer aufweist, die beide in einer gemeinsamen Auslassöffnung (C) enden. Der Kopfbereich ist derart gestaltet, dass die beiden Zubereitungen gemeinsam aus der Tube austreten, sobald Druck auf diese ausgeübt wird. Durch die Gestaltung dieses Kopfbereiches wird bestimmt, in welchem Muster die Zubereitungen aus der Tube austreten. Prinzipiell kann die vorliegende Erfindung jede Verteilung der Kammern innerhalb der Tube umfassen. In einer ersten Ausführungsform können beispielsweise die beiden einzelnen Kammern nebeneinander in einer äußeren Hülle angeordnet sein. In einer erfindungsgemäß besonders bevorzugten Ausführungsform besteht die Mehrkammertube jedoch aus einer inneren Tube, die von einer äußeren Tube vollständig umgeben ist.

Die Mehrkammertube ist vorzugsweise aus einem Material gefertigt, das zur Verpackung von oxdiativen Farbänderungsmitteln dieser Art geeignet ist. Als erfindungsgemäß besonders geeignet hat sich sowohl für die Außenwände als auch für die Innenwände laminiertes Aluminium erwiesen. Es sind aber auch Tuben aus Kunststofflaminat (PE, PET, PP) oder Kunststoffcoextrudaten (PE, PET, PP) denkbar. Darüber hinaus kann in einer Ausführungsform das Material der Innentube unabhängig von dem Material der Außentube gewählt werden. Als erfindungsgemäß ganz besonders bevorzugt hat sich eine Tube erwiesen, bei der die Innentube aus Aluminiumlaminat, das gegebenenfalls noch mit einem Lack geschützt ist, und die Außentube entweder aus Aluminiumlaminat oder aus Kunststofflaminat gefertigt ist. Unter Aluminiumlaminat wird erfindungsgemäß eine mit Kunststoff beschichtete Aluminiumschicht verstanden.

Es ist besonders vorteilhaft, wenn der Schulterbereich der Außentube mit Ronden verstärkt wird, die besonders gute Barriereeigenschaften aufweisen. Dabei ist es vorteilhaft, in das Material der Ronden Aluminium einzuarbeiten. Um ein Austreten der Mischung während der Lagerung zu verhindern und dem Verbraucher die Unversehrtheit der Tube zu versichern, ist es vorteilhaft, die Ausgabeöffnung mit einem Originalitätsverschluss aus Aluminium oder Kunststoff

zu versiegeln, der vom Verbraucher entfernt wird.

Die Wahl der Volumina der einzelnen Kammern (A) und (B) richtet sich nach dem gewünschten Verhältnis der Volumina von Zubereitung (a) und Zubereitung (b). Bevorzugt sind die Volumina der Kammern (A) und (B) gleich. Das Mengenverhältnis der Zubereitung (a) zur Menge der Zubereitung (b) liegt erfindungsgemäß bevorzugt in einem Bereich von 1:3 bis 3:1, ein Bereich von 1:1,5 bis 1,5:1 ist erfindungsgemäß bevorzugt, besonders bevorzugt ist ein Mengenverhältnis von 1:1.

Ebenfalls ganz besonders bevorzugt ist ein Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- einen oder mehrere der genannten verdickenden Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- einen oder mehrere der genannten verdickenden Polymere in einer Gesamtgewichtsmenge (G2) von 0,1 bis 40 Gew.-% - bezogen auf das Gesamtgewicht der Zubereitung (b),
  dadurch gekennzeichnet, dass
- das Gewichtsverhältnis G1/G2 bei einem Wert von 0,5 bis 2,0 liegt und
- das Gewichtsverhältnis aus dem Gesamtgewicht der Zubereitung (a) zum Gesamtgewicht der Zubereitung (b) bei einem Wert von 0,5 bis 2,0, bevorzugt von 0,6 bis 1,8, weiter bevorzugt von 0,7 bis 1,4 und besonders bevorzugt von 0,8 bis 1,2 liegt.

Die erfindungsgemäßen Produkte können in Verfahren zur oxidativen Änderung der Haarfarbe eingesetzt werden. Diese Verfahren zeichnen sich für den Verbraucher durch besonderen Anwendungkomfort aus, da die aufwändige und fehleranfällige Herstellung der Anwendungsmischungen durch den Verbraucher entfällt. Zudem wird mit Anwendung der Produkte ein besonders gleichmäßiges Farbergebnis ermöglicht, da es durch die spezielle rheologische Angleichung der Formulierungen (a) und (b) möglich wird, die beiden Formulierungen während des gesamten Applikationsprozesses in genau definierten, zueinander stets gleichen Mengenanteilen zu entnehmen.

Die Entnahme der Anwendungsmischung erfolgt hierbei üblicherweise portionsweise, so dass die Kammern (A) und (B) des Produktes schrittweise in definiertem Verhältnis zueinander entleert werden. Im Regelfall wird der Verbraucher die für die Färbung bzw. Aufhellung einer oder mehrerer Strähnen bzw. Haarpartien notwendige Menge der Anwendungsmischung aus dem Spender entnehmen, und diese dann auf die Haare applizieren. Diesen Vorgang wird der Verbraucher solange wiederholen, bis entweder das Produkt vollständig entleert ist oder bis der Verbraucher alle Haare behandelt hat.

Insbesondere bei Konfektionierung des Produktes in Form eines Druckbehälters ist jedoch auch eine kontinuierliche Entnahme des Produktes denkbar, d.h. in diesem Fall werden die Kammern (A) und (B) durch Betätigung der Auslassöffnung (C) kontinuierlich entleert, wobei auch hier die Zusammensetzung der Anwendungsmischung (aus den Zubereitungen (a) und (b)) zu jedem Zeitpunkt der Entnahme gleich und definiert ist.

Ein weiterer Gegenstand der vorliegenden Erfindung ist ein Verfahren zum Färben und/oder Aufhellen von keratinischen Fasern unter Anwendung eines Produktes des ersten Erfindungsgegenstands, wobei

- der Spender kontinuierlich in einem Schritt oder portionsweise in einzelnen Schritten entleert wird und

- bei jedem Schritt jeweils definierte, konstante Mengen der Zubereitungen (a) und (b) aus den Kammern (A) und (B) über die gemeinsame Auslassöffnung (C) des Spenders bis zur vollständigen Entleerung der beiden Kammern (A) und (B) entnommen werden.

Bevorzugt ist ein Verfahren, bei welchem die Kammern (A) und (B) portionsweise in mehreren Schritten entleert werden.

Besonders bevorzugt ist ein Verfahren zum Färben und/oder Aufhellen von keratinischen Fasern unter Anwendung eines Produktes des ersten Erfindungsgegenstands, wobei

- der Spender portionsweise in mehreren Schritten entleert wird und
- bei jedem Schritt jeweils definierte, konstante Mengen der Zubereitungen (a) und (b) aus den Kammern (A) und (B) über die gemeinsame Auslassöffnung (C) des Spenders bis zur vollständigen Entleerung der beiden Kammern (A) und (B) entnommen werden.

In diesem Zusammenhang kann es erwünscht sein, das bei jedem Schritt bzw. jeweils gleiche Mengen der Zubereitungen (a) und (b) entnommen werden. Besitzt eine der Kammern (A) oder (B) jedoch ein größeres Volumen bzw. Fassungsvermögen und/oder ist eine der Zubereitungen (a) und (b) in größerer Menge in dem Produkt enthalten, so kann es auch gewünscht sein, die Zubereitungen (a) und (b) bei jedem Schritt in einem jeweils konstanten Mengenverhältnis von 3:1 bis 1:3 zu entnehmen.

Besonders bevorzugt ist es, wenn die Menge der schrittweise entnommenen Zubereitungen (a) und (b) gleich ist, d.h. wenn bei jedem Entnahmeschritt die gleichen Mengen (a) und (b) dem Spender entnommen werden.

Ganz besonders bevorzugt ist daher ein Verfahren zum Färben und/oder Aufhellen von keratinischen Fasern, welches dadurch gekennzeichnet ist, dass

- bei jedem Schritt jeweils gleiche Mengen der Zubereitungen (a) und (b) aus den Kammern (A) und (B) über die gemeinsame Auslassöffnung (C) des Spenders bis zur vollständigen Entleerung der beiden Kammern (A) und (B) entnommen werden.

Bezüglich weiterer bevorzugter Ausführungsformen des erfindungsgemäßen Verfahrens gilt mutatis mutandis das zu dem erfindungsgemäßen Produkt Gesagte.

Beispiele

1. Herstellung der Zubereitungen (a) und (b)

Es wurden die folgenden Zubereitungen (a) und (b) hergestellt (alle Angaben in Gew.-% Aktivsubstanz):

| **Inhaltsstoffe** | | **Zubereitung (a)** | **Zubereitung (b)** |
|---|---|---|---|
| Xanthan Gum | verdickendes Polymer | 2,00 | 2,00 |
| PEG-60 Hydrogenated Castor Oil | nichtionischer Emulgator | 0,3 | 0,3 |
| p-Toluylendiamin, Sulfat ($C_7H_{12}N_2^{2+}$ x $SO_4^{2-}$) (molare Masse = 220,25 g/mol); | OFV Salz | 0,111 (5,04 x $10^{-3}$ mol/kg) | -- |
| Resorcin | OFV | 0,03 | --- |
| m-Aminophenol | OFV | 0,002 | --- |
| 4-Chlorresorcin | OFV | 0,03 | --- |
| 2-Methylresorcin | OFV | 0,01 | --- |
| Ammoniumsulfat $(NH_4)_2SO_4$ (molare Masse = 132,13 g/mol) | Salz | 0,93 (0,070 mol/kg) | --- |
| Natriumsulfit $Na_2SO_3$ (molare Masse = 126,04 g/mol) | Salz | 0,40 (0,0317 mol/kg) | --- |
| Vitamin C | Reduktionsmittel | 0,10 | --- |
| Hydroxyethan-1,1-diphosphonsäure, Dikaliumsalz (molare Masse = 282,27 g/mol) | Salz | --- | 0,9 (0,032 mol/kg) |
| Ammoniak (NH3) | Alkalisierungsmittel | 2,00 | --- |
| Propylenglykol | LSM | 4,00 | -- |
| Dipicolinsäure, Monokaliumsalz (molare Masse = 205,20 g/mol) | Salz | --- | 0,10 (4,87 x $10^{-3}$ mol/kg) |

(fortgesetzt)

| Inhaltsstoffe | | Zubereitung (a) | Zubereitung (b) |
|---|---|---|---|
| Dinatriumpyrophosphat ($H_2O_7P_2Na_2$) (molare Masse = 222,053 g/mol) | Salz | --- | 0,03 (1,35 x $10^{-3}$ mol/kg) |
| Natriumchlorid NaCl (molare Masse = 58,44 g/mol) | Salz | --- | 0,40 (0,0685 mol/kg) |
| Wasserstoffperoxid | OX | --- | 6,0 |
| Wasser (dest) | | ad 100 | ad 100 |
| Viskosität (22 °C / Brookfield-Viskosimeter / Spindel 5 / 4 Upm) | | 20 000 mPas | 20 000 mPas |

Abkürzungen:

OFV: Oxidationsfarbstoffvorprodukt
LSM: Lösungsmittel
OX: Oxidationsmittel

2. Gesamtgewichtsverhältnis G1/G2

| | Zubereitung (a) G1 | Zubereitung (b) G2 |
|---|---|---|
| Xanthan Gum | 2,00 | 2,00 |
| Verhältnis G1/G2 | 1,0 | |

3. Gesamtmolalitäten der Salze in den Zubereitungen (a) und (b)

| | Zubereitung (a) M1 | Zubereitung (b) M2 |
|---|---|---|
| Gesamtmolalitäten Salze | 0,107 mol/kg | 0,107 |
| Verhältnis M1/M2 | 1,0 | |

**Patentansprüche**

**1.** Produkt zur oxidativen Färbung und/oder Aufhellung keratinischer Fasern, umfassend

(1) einen Spender, der

- zwei voneinander getrennte Kammern (A) und (B) aufweist,
- eine Auslassöffnung (C) aufweist, die mit der Kammer (A) und mit der Kammer (B) in Verbindung steht,

(2) eine Zubereitung (a) in der Kammer (A), enthaltend in einem wässrigen, kosmetischen Träger

- ein oder mehrere verdickende Polymere in einer Gesamtgewichtsmenge (G1) von 0,1 bis 40 Gew.-% - bezogen auf das Gesamtgewicht der Zubereitung (a),

(3) eine Zubereitung (b) in der Kammer (B), enthaltend in einem wässrigen, kosmetischen Träger

- ein oder mehrere verdickende Polymere in einer Gesamtgewichtsmenge (G2) von 0,1 bis 40 Gew.-% - bezogen auf das Gesamtgewicht der Zubereitung (b),
**dadurch gekennzeichnet, dass**
- das Gewichtsverhältnis G1/G2 bei einem Wert von 0,5 bis 2,0 liegt und
- die Zubereitung (a) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, enthält und
- die Zubereitung (b) als verdickende(s) Polymer(e) ein oder mehrere Polysaccharide aus der Gruppe der Carboxymethylcellulosen, der Alginsäuren, Xanthan Gum und/oder deren physiologisch verträglichen Salzen, enthält.

**2.** Produkt nach Anspruch 1, **dadurch gekennzeichnet, dass** das Gewichtsverhältnis von G1/G2 bei einem Wert von, bevorzugt von 0,55 bis 1,8, weiter bevorzugt von 0,6 bis 1,6 und besonders bevorzugt von 0,7 bis 1,4 liegt.

**3.** Produkt nach einem der Ansprüche 1 bis 2, **dadurch gekennzeichnet, dass**

- die Zubereitung (a) als verdickende(s) Polymer(e) Xanthan Gum und/oder dessen physiologisch verträgliche Salze enthält und
- die Zubereitung (b) als verdickende(s) Polymer(e) Xanthan Gum und/oder dessen physiologisch verträgliche Salze enthält.

**4.** Produkt nach einem der Ansprüche 1 bis 3, **dadurch gekennzeichnet, dass**

- die Zubereitung (a) das oder die verdickenden Polymere in einer Gesamtgewichtsmenge (G1) von 0,2 bis 9,5 Gew.-%, bevorzugt von 0,4 bis 7,5 Gew.-%, weiter bevorzugt von 0,6 bis 5,5 Gew.-% und besonders bervorzugt von 0,8 bis 2,5 Gew.-% - bezogen auf das Gesamtgewicht der Zubereitung (a) - enthält und
- die Zubereitung (b) das oder die verdickende Polymere in einer Gesamtgewichtsmenge (G2) von 0,2 bis 9,5 Gew.-%, bevorzugt von 0,4 bis 7,5 Gew.-%, weiter bevorzugt von 0,6 bis 5,5 Gew.-% und besonders bevorzugt von 0,8 bis 2,5 Gew.-% - bezogen auf das Gesamtgewicht der Zubereitung (b) - enthält.

**5.** Produkt nach einem der Ansprüche 1 bis 4, **dadurch gekennzeichnet, dass**

- die Zubereitung (a) eine Viskosität von 1000 bis 100 000 mPas, bevorzugt von 2000 bis 80 000 mPas, weiter bevorzugt von 4000 bis 60 000 mPas, noch weiter bevorzugt von 7000 bis 50 000 mPas und besonders bevorzugt von 10 000 bis 40 000 mPas besitzt
(22 °C / Brookfield-Viskosimeter / Spindel 5/4 Upm),
- die Zubereitung (b) eine Viskosität von 1000 bis 100 000 mPas, bevorzugt von 2000 bis 80 000 mPas, weiter bevorzugt von 4000 bis 60 000 mPas, noch weiter bevorzugt von 7000 bis 50 000 mPas und besonders bevorzugt von 10 000 bis 40 000 mPas besitzt
(22 °C / Brookfield-Viskosimeter / Spindel 5 / 4 Upm) und
- das Verhältnis der Viskositäten V1/V2 bei einem Wert von 0,3 bis 3,0, bevorzugt von 0,4 bis 2,5, und besonders bevorzugt von 0,5 bis 2,0 liegt.

**6.** Produkt nach einem der Ansprüche 1 bis 5, **dadurch gekennzeichnet, dass**

- die Zubereitung (a) zusätzlich ein oder mehrere organische und/oder anorganische Salze
in einer Gesamtmolalität (M1) von 0,01 bis 1 mol/kg - bezogen auf das Gesamtgewicht der Zubereitung (a) enthält
- die Zubereitung (b) zusätzlich ein oder mehrere organische und/oder anorganische Salze
in einer Gesamtmolalität (M2) von 0,01 bis 1 mol/kg - bezogen auf das Gesamtgewicht der Zubereitung (b) enthält und
- das Molalitätsverhältnis M1/M2 bei einem Wert von 0,3 bis 3,0 liegt.

**7.** Produkt nach Anspruch 6, **dadurch gekennzeichnet, dass** das Molalitätsverhältnis M1/M2 bei einem Wert von 0,4 bis 2,5, bevorzugt von 0,5 bis 2,0, weiter bevorzugt von 0,6 bis 1,7, und besonders bevorzugt von 0,7 bis 1,4 liegt.

**8.** Produkt nach einem der Ansprüche 1 bis 7, **dadurch gekennzeichnet, dass**

- die Zubereitung (a) ein oder mehrere organische und/oder anorganische Salze in einer Ionenstärke (I1) enthält, die sich nach der Formel (1) berechnet

$$I1 = \frac{1}{2}\sum_{i} z_{i}^{2} ci \qquad (1)$$

mit zi gleich der Ladungszahl jedes in der Zubereitung (a) enthaltenen Ions (i) und
ci gleich der Molalität (mol/kg) jedes in der Zubereitung (a) enthaltenen Ions, wobei sich die Molalität auf das Gesamtgewicht der Zubereitung (a) bezieht,
- die Zubereitung (b) ein oder mehrere organische und/oder anorganische Salze in einer Ionenstärke (I2) enthält, die sich nach der Formel (2) berechnet

$$I2 = \frac{1}{2}\sum_{i} z_{i}^{2} ci \qquad (2)$$

mit zi gleich der Ladungszahl jedes in der Zubereitung (b) enthaltenen Ions (i) und
ci gleich der Molalität (mol/kg) jedes in der Zubereitung (b) enthaltenen Ions, wobei sich die Molalität auf das Gesamtgewicht der Zubereitung (b) bezieht, und
- das Verhältnis der Ionenstärken I1/I2 bei einem Wert von 0,5 bis 2,0, bevorzugt von 0,6 bis 1,8, weiter bevorzugt von 0,7 bis 1,6 liegt.

**9.** Produkt nach einem der Ansprüche 1 bis 8, **dadurch gekennzeichnet, dass**

- die Zubereitung (a) mindestens ein Alkalisierungsmittel, bevorzugt Ammoniak und/oder Monoethanolamin, und
- die Zubereitung (b) mindestens ein Oxidationsmittel, bevorzugt Wasserstoffperoxid, enthält.

**10.** Produkt nach einem der Ansprüche 1 bis 9, **dadurch gekennzeichnet, dass**

- die Zubereitung (a) einen pH-Wert von 8 bis 12,5, bevorzugt von 8,5 bis 12, weiter bevorzugt von 9 bis 11,5 und besonders bevorzugt von 9,5 bis 11 besitzt
- die Zubereitung (b) einen pH-Wert von 1 bis 7, bevorzugt von 1,5 bis 6, weiter bevorzugt von 2,5 bis 5,5 und besonders bevorzugt von 3,0 bis 5,0 besitzt.

**11.** Produkt nach einem der Ansprüche 1 bis 10, **dadurch gekennzeichnet, dass**

- es sich bei dem Spender um einen Druckbehälter handelt
- es sich bei der Auslassöffnung (C) um eine Druckbehälter-Abgabevorrichtung handelt und
- der Spender zusätzlich mindestens ein Treibgas aus der Gruppe Propan, Propen, n-Butan, iso-Butan, iso-Buten, n-Pentan, Penten, iso-Pentan, iso-Penten, Luft, Stickstoff, Argon, $N_2O$ und/oder $CO_2$ enthält

**12.** Produkt nach einem der Ansprüche 1 bis 10, **dadurch gekennzeichnet, dass**

- es sich bei dem Spender um einen Spender für pastöse Massen, eine Tube oder einen Quetschbehälter handelt und
- es sich bei der Auslassöffnung (C) um ein Pumpelement, ein Ventil oder eine Abgabevorrichtung für pastöse Massen handelt.

**13.** Verfahren zum Färben und/oder Aufhellen von keratinischen Fasern unter Anwendung eines Produktes nach einem der Ansprüche 1 bis 12, wobei

- der Spender kontinulierlich in einem Schritt oder portionsweise in einzelnen Schritten entleert wird und
- bei jedem Schritt jeweils definierte, konstante Mengen der Zubereitungen (a) und (b) aus den Kammern (A) und (B) über die gemeinsame Auslassöffnung (C) des Spenders bis zur vollständigen Entleerung der beiden Kammern (A) und (B) entnommen werden.

**14.** Verfahren nach Anspruch 13, **dadurch gekennzeichnet, dass**

- bei jedem Schritt jeweils gleiche Mengen der Zubereitungen (a) und (b) aus den Kammern (A) und (B) über die gemeinsame Auslassöffnung (C) des Spenders bis zur vollständigen Entleerung der beiden Kammern (A) und (B) entnommen werden.

## Claims

1. A product for oxidatively dyeing and/or lightening keratin fibers, comprising

(1) a dispenser, which comprises

- two separate chambers (A) and (B),
- an outlet opening (C), which is connected to chamber (A) and to chamber (B),

(2) a preparation (a) in chamber (A), containing, in an aqueous cosmetic carrier,

- one or more thickening polymers in a total amount by weight (G1) of from 0.1 to 40 wt.%, based on the total weight of preparation (a),

(3) a preparation (b) in chamber (B), containing, in an aqueous cosmetic carrier,

- one or more thickening polymers in a total amount by weight (G2) of from 0.1 to 40 wt.%, based on the total weight of preparation (b),

**characterized in that**

- the weight ratio G1/G2 is a value of from 0.5 to 2.0 and
- preparation (a) contains, as the thickening polymer(s), one or more polysaccharides from the group of carboxymethyl celluloses, alginic acids, xanthan gum and/or the physiologically acceptable salts thereof, and
- preparation (b) contains, as the thickening polymer(s), one or more polysaccharides from the group of carboxymethyl celluloses, alginic acids, xanthan gum and/or the physiologically acceptable salts thereof.

2. The product according to claim 1, **characterized in that** the weight ratio of G1/G2 is a value preferably of from 0.55 to 1.8, more preferably from 0.6 to 1.6 and particularly preferably from 0.7 to 1.4.

3. The product according to one of claims 1 to 2, **characterized in that**
preparation (a) contains, as the thickening polymer(s), xanthan gum and/or the physiologically acceptable salts thereof, and
preparation (b) contains, as the thickening polymer(s), xanthan gum and/or the physiologically acceptable salts thereof.

4. The product according to one of claims 1 to 3, **characterized in that**

- preparation (a) contains the thickening polymer or thickening polymers in a total amount by weight (G1) of from 0.2 to 9.5 wt.%, preferably from 0.4 to 7.5 wt.%, more preferably from 0.6 to 5.5 wt.% and particularly preferably from 0.8 to 2.5 wt.%, based on the total weight of preparation (a), and
- preparation (b) contains the thickening polymer or thickening polymers in a total amount by weight (G2) of from 0.2 to 9.5 wt.%, preferably from 0.4 to 7.5 wt.%, more preferably from 0.6 to 5.5 wt.% and particularly preferably from 0.8 to 2.5 wt.%, based on the total weight of preparation (b).

5. The product according to one of claims 1 to 4, **characterized in that**

- preparation (a) has a viscosity of from 1,000 to 100,000 mPas, preferably from 2,000 to 80,000 mPas, more preferably from 4,000 to 60,000 mPas, even more preferably from 7,000 to 50,000 mPas and particularly preferably from 10,000 to 40,000 mPas (22 °C / Brookfield viscometer / spindle 5 / 4 rpm),
- preparation (b) has a viscosity of from 1,000 to 100,000 mPas, preferably from 2,000 to 80,000 mPas, more preferably from 4,000 to 60,000 mPas, even more preferably from 7,000 to 50,000 mPas and particularly preferably from 10,000 to 40,000 mPas (22 °C / Brookfield viscometer / spindle 5 / 4 rpm), and
- the ratio of the viscosities V1/V2 is a value of from 0.3 to 3.0, preferably from 0.4 to 2.5 and particularly preferably from 0.5 to 2.0.

**6.** The product according to one of claims 1 to 5, **characterized in that**

- preparation (a) also contains one or more organic and/or inorganic salts in an overall molality (M1) of from 0.01 to 1 mol/kg, based on the total weight of preparation (a)
- preparation (b) also contains one or more organic and/or inorganic salts in an overall molality (M2) of from 0.01 to 1 mol/kg, based on the total weight of preparation (b) and
- the molality ratio M1/M2 is a value of from 0.3 to 3.0.

**7.** The product according to claim 6, **characterized in that** the molality ratio M1/M2 is a value of from 0.4 to 2.5, preferably from 0.5 to 2.0, more preferably from 0.6 to 1.7, and particularly preferably from 0.7 to 1.4.

**8.** The product according to one of claims 1 to 7, **characterized in that**

- preparation (a) contains one or more organic and/or inorganic salts having an ionic strength (11) calculated according to formula (1)

$$I1 = \frac{1}{2}\sum_i z_i^2 ci \qquad (1)$$

where zi is equal to the charge number of each ion (i) contained in preparation (a) and ci is equal to the molality (mol/kg) of each ion contained in preparation (a), the molality being based on the total weight of preparation (a),
- preparation (b) contains one or more organic and/or inorganic salts having an ionic strength (12) calculated according to formula (2)

$$I2 = \frac{1}{2}\sum_i z_i^2 ci \qquad (2)$$

where zi is equal to the charge number of each ion (i) contained in preparation (b) and ci is equal to the molality (mol/kg) of each ion contained in preparation (b), the molality being based on the total weight of preparation (b), and
- the ratio of ionic strengths I1/I2 is a value of from 0.5 to 2.0, preferably from 0.6 to 1.8, more preferably from 0.7 to 1.6.

**9.** The product according to one of claims 1 to 8, **characterized in that**

- preparation (a) contains at least one alkalizing agent, preferably ammonia and/or monoethanolamine, and
- preparation (b) contains at least one oxidizing agent, preferably hydrogen peroxide.

**10.** The product according to one of claims 1 to 9, **characterized in that**

- preparation (a) has a pH of from 8 to 12.5, preferably from 8.5 to 12, more preferably from 9 to 11.5, and particularly preferably from 9.5 to 11
- preparation (b) has a pH of from 1 to 7, preferably from 1.5 to 6, more preferably from 2.5 to 5.5 and particularly preferably from 3.0 to 5.0.

**11.** The product according to one of claims 1 to 10, **characterized in that**

- the dispenser is a pressurized container
- the outlet opening (C) is a pressurized container dispensing device and
- the dispenser also contains at least one propellant gas from the group of propane, propene, n-butane, iso-butane, iso-butene, n-pentane, pentene, iso-pentane, isopentene, air, nitrogen, argon, $N_2O$ and/or $CO_2$.

**12.** The product according to one of claims 1 to 10, **characterized in that**

- the dispenser is a dispenser for pasty materials, a tube or a squeeze container and
- the outlet opening (C) is a pump element, a valve or a dispensing device for pasty materials.

**13.** A method for dyeing and/or lightening keratin fibers by applying a product according to one of claims 1 to 12, wherein

- the dispenser is emptied continuously in one step or in portions in individual steps and
- in each step, defined, constant amounts of preparations (a) and (b) are removed from chambers (A) and (B) via the common outlet opening (C) of the dispenser until the two chambers (A) and (B) are completely empty.

**14.** The method according to claim 13, **characterized in that**

- in each step, identical amounts of preparations (a) and (b) are removed from chambers (A) and (B) via the common outlet opening (C) of the dispenser until the two chambers (A) and (B) are completely empty.

## Revendications

**1.** Produit de coloration par oxydation et/ou de décoloration de fibres de kératine, comprenant :

(1) un distributeur qui comporte

- deux chambres (A) et (B) séparées l'une de l'autre,
- un orifice de sortie (C) en relation avec la chambre (A) et la chambre (B),

(2) une préparation (a) dans la chambre (A) contenant, dans un support cosmétique aqueux

- au moins un polymère épaississant dans une quantité totale en poids (G1) de 0,1 à 40 % en poids - rapportée au poids total de la préparation (a),

(3) une préparation (b) dans la chambre (B) contenant, dans un support cosmétique aqueux

- au moins un polymère épaississant dans une quantité totale en poids (G2) de 0,1 à 40 % en poids - rapportée au poids total de la préparation (b),
**caractérisé en ce que**
- le rapport en poids G1/G2 est entre 0,5 et 2,0, et
- la préparation (a) contient comme polymère(s) épaississant(s) au moins un polysaccharide du groupe des carboxyméthylcelluloses, des acides alginiques, de la gomme xanthane et/ou de leurs sels physiologiquement compatibles, et
- la préparation (b) contient comme polymère(s) épaississant(s) au moins un polysaccharide du groupe des carboxyméthylcelluloses, des acides alginiques, de la gomme xanthane et/ou de leurs sels physiologiquement compatibles.

**2.** Produit selon la revendication 1, **caractérisé en ce que** le rapport en poids G1/G2 est préférentiellement de 0,55 à 1,8, plus préférentiellement de 0,6 à 1,6 et particulièrement préférentiellement de 0, 7 à 1,4.

**3.** Produit selon une des revendications 1 à 2, **caractérisé en ce que** la préparation (a) contient comme polymère(s) épaississant(s) de la gomme xanthane et/ou ses sels physiologiquement compatibles, et
la préparation (b) contient comme polymère(s) épaississant(s) de la gomme xanthane et/ou ses sels physiologiquement compatibles.

**4.** Produit selon une des revendications 1 à 3, **caractérisé en ce que**

- la préparation (a) contient le ou les polymères épaississants dans une quantité totale en poids (G1) de 0,2 à 9,5 % en poids, de préférence de 0,4 à 7,5 % en poids, plus préférentiellement de 0,6 à 5,5 % en poids et particulièrement préférentiellement de 0,8 à 2,5 % en poids - rapporté au poids total de la préparation (a)
- la préparation (b) contient le ou les polymères épaississants dans une quantité totale en poids (G2) de 0,2 à 9,5 % en poids, de préférence de 0,4 à 7,5 % en poids, plus préférentiellement de 0,6 à 5,5 % en poids et particulièrement préférentiellement de 0,8 à 2,5 % en poids - rapporté au poids total de la préparation (b).

**5.** Produit selon une des revendications 1 à 4, **caractérisé en ce que**

- la préparation (a) a une viscosité de 1 000 à 100 000 mPa.s, de préférence de 2 000 à 80 000 mPa.s, plus préférentiellement de 4 000 à 60 000 mPa.s, encore plus préférentiellement de 7 000 à 50 000 mPa.s et particulièrement préférentiellement de 10 000 à 40 000 mPa.s (22°C / viscosimètre Brookfield / broche 5/4 tr/min),
- la préparation (b) a une viscosité de 1 000 à 100 000 mPa.s, de préférence de 2 000 à 80 000 mPa.s, plus préférentiellement de 4 000 à 60 000 mPa.s, encore plus préférentiellement de 7 000 à 50 000 mPa.s et particulièrement préférentiellement de 10 000 à 40 000 mPa.s (22°C / viscosimètre Brookfield / broche 5/4 tr/min),
- le rapport des viscosités V1/V2 est de 0,3 à 3,0, de préférence de 0,4 à 2,5 et particulièrement préférentiellement de 0,5 à 2,0.

**6.** Produit selon une des revendications 1 à 5, **caractérisé en ce que**

- la préparation (a) contient en plus au moins un sel organique et/ou minéral dans une molalité totale (M1) de 0,01 à 1 mol/kg - rapportée au poids total de la préparation (a)
- la préparation (b) contient en plus au moins un sel organique et/ou minéral dans une molalité totale (M2) de 0,01 à 1 mol/kg - rapportée au poids total de la préparation (b) et
- le rapport de molalité M1/M2 est de 0,3 à 3,0.

**7.** Produit selon la revendication 6, **caractérisé en ce que** le rapport de molalité M1/M2 est de 0,4 à 2;5, de préférence de 0,5 à 2,0, plus préférentiellement de 0,6 à 1,7, et particulièrement préférentiellement de 0, 7 à 1,4.

**8.** Produit selon une des revendications 1 à 7 **caractérisé en ce que**

- la préparation (a) contient au moins un sel organique et/ou minéral avec une force ionique (11) qui se calcule selon la formule (1)

$$I1 = \frac{1}{2}\sum_i z_i^2 ci \qquad (1)$$

où

$z_i$ est égal au nombre de charge de chaque ion (i) contenu dans la préparation (a), et
$c_i$ est égal à la molalité (mol/kg) de chaque ion contenu dans la préparation (a), la molalité se rapportant au poids total de la préparation (a),

- la préparation (b) contient au moins un sel organique et/ou minéral avec une force ionique (12) qui se calcule selon la formule (2)

$$I2 = \frac{1}{2}\sum_i z_i^2 ci \qquad (2)$$

où

$z_i$ est égal au nombre de charge de chaque ion (i) contenu dans la préparation (b), et
$c_i$ est égal à la molalité (mol/kg) de chaque ion contenu dans la préparation (b), la molalité se rapportant au poids total de la préparation (b), et

- le rapport des forces ioniques 11/12 est entre 0,5 à 2,0, de préférence de 0,6 à 1,8, plus préférentiellement de 0,7 à 1,6.

**9.** Produit selon une des revendications 1 à 8, **caractérisé en ce que**

- la préparation (a) contient au moins un agent d'alcalisation, de préférence l'ammoniac et/ou la monoéthanolamine, et
- la préparation (b) contient un oxydant, de préférence du peroxyde d'hydrogène.

**10.** Produit selon une des revendications 1 à 9. **caractérisé en ce que**

- la préparation (a) a un pH de 8 à 12,5, de préférence de 8,5 à 12, plus préférentiellement de 9 à 11,5 et particulièrement préférentiellement de 9,5 à 11
- la préparation (b) a un pH de 1 à 7, de préférence de 1,5 à 6, plus préférentiellement de 2,5 à 5,5 et particulièrement préférentiellement de 3,0 à 5,0.

**11.** Produit selon une des revendications 1 à 10, **caractérisé en ce que**

- il s'agit d'un distributeur et d'un réservoir sous pression
- l'orifice de sortie (C) est un distributeur à réservoir sous pression, et
- le distributeur contient, en plus, au moins un gaz vecteur issu du groupe constitué du propane, du propène, du n-butane, de l'isobutane, de l'isobutène, du n-pentane, du pentène, de l'isopentane, de l'isopentène, de l'air, de l'azote, de l'argon, de $N_2O$ et/ou du $CO_2$.

**12.** Produit selon une des revendications 1 à 10, **caractérisé en ce que**

- le distributeur est un distributeur de masses pâteuses, un tube ou un récipient à presser,
- l'orifice de sortie (C) est un élément de pompe, une soupape ou un dispositif de distribution de masses pâteuses.

**13.** Procédé de coloration et/ou d'éclaircissement de fibres de kératine par utilisation d'un produit selon une des revendications 1 à 12, où

- le distributeur est vidé en continu en une seule étape ou par portions en plusieurs étapes, et
- à chaque étape, des quantités constantes et définies, des préparations (a) et (b) sont prélevées respectivement des chambres (A) et (B) par l'orifice de sortie commune (C) du distributeur jusqu'à vidange complète des deux chambres (A) et (B).

**14.** Procédé selon la revendication 13, **caractérisé en ce que**

- à chaque étape, des quantités égales des préparations (a) et (b) sont prélevées respectivement des chambres (A) et (B) par l'orifice de sortie commune (C) du distributeur jusqu'à vidange complète des deux chambres (A) et (B).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102007056935 A1
- DE 3729491 A1
- US 20090108021 A1